(19) Europäisches Patentamt
European Patent Office
Office européen des brevets

(11) EP 3 193 383 B1

(12) EUROPEAN PATENT SPECIFICATION

(45) Date of publication and mention of the grant of the patent:
**23.02.2022 Bulletin 2022/08**

(21) Application number: **15837910.7**

(22) Date of filing: **26.08.2015**

(51) International Patent Classification (IPC):
***H01L 51/30*** *(2006.01)* ***H01L 21/368*** *(2006.01)*
***H01L 29/786*** *(2006.01)* ***H01L 51/05*** *(2006.01)*
***H01L 51/40*** *(2006.01)* ***C07C 43/162*** *(2006.01)*
***C07D 307/77*** *(2006.01)* ***C07D 333/18*** *(2006.01)*
***C07D 493/06*** *(2006.01)* ***C07D 495/04*** *(2006.01)*
***C07D 495/14*** *(2006.01)* ***C07D 495/22*** *(2006.01)*
***C09D 165/00*** *(2006.01)* ***C09D 5/24*** *(2006.01)*
***H01B 1/12*** *(2006.01)* ***H01B 1/20*** *(2006.01)*

(52) Cooperative Patent Classification (CPC):
(C-Sets available)
**H01L 51/0074; C07D 333/18; C07D 493/06; C07D 495/04; C07D 495/14; C07D 495/22; C07F 7/00; C08K 5/01; C08K 5/45; C09D 5/24; C09D 11/106; C09D 11/36; C09D 11/52; C09D 165/00; H01L 29/786;** (Cont.)

(86) International application number:
**PCT/JP2015/074027**

(87) International publication number:
**WO 2016/035640 (10.03.2016 Gazette 2016/10)**

(54) **COMPOSITION FOR FORMING ORGANIC SEMICONDUCTOR FILM, ORGANIC SEMICONDUCTOR FILM AND METHOD FOR MANUFACTURING SAME, AND ORGANIC SEMICONDUCTOR COMPOUND**

ZUSAMMENSETZUNG ZUR HERSTELLUNG EINER ORGANISCHEN HALBLEITERFOLIE, ORGANISCHE HALBLEITERFOLIE UND VERFAHREN ZUR HERSTELLUNG DAVON SOWIE ORGANISCHE HALBLEITERVERBINDUNG

COMPOSITION DE FORMATION DE FILM DE SEMI-CONDUCTEUR ORGANIQUE, FILM DE SEMI-CONDUCTEUR ORGANIQUE ET SON PROCÉDÉ DE FABRICATION, ET ÉLÉMENT DE SEMI-CONDUCTEUR ORGANIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.09.2014 JP 2014177324**

(43) Date of publication of application:
**19.07.2017 Bulletin 2017/29**

(73) Proprietor: **FUJIFILM Corporation**
**Tokyo 106-8620 (JP)**

(72) Inventors:
- **KITAMURA, Tetsu**
  **Ashigarakami-gun**
  **Kanagawa 258-8577 (JP)**
- **KOYANAGI, Masashi**
  **Ashigarakami-gun**
  **Kanagawa 258-8577 (JP)**
- **SHIGENOI, Yuta**
  **Ashigarakami-gun**
  **Kanagawa 258-8577 (JP)**

(74) Representative: **Hoffmann Eitle**
**Patent- und Rechtsanwälte PartmbB**
**Arabellastraße 30**
**81925 München (DE)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 193 383 B1

(56) References cited:
**JP-A- 2007 158 062** **JP-A- 2009 267 134**
**JP-A- 2009 267 140** **JP-A- 2010 177 642**
**JP-A- 2011 142 145** **JP-A- 2013 544 755**
**JP-A- 2014 122 189** **JP-B2- 5 352 260**
**US-A- 5 936 259**

(52) Cooperative Patent Classification (CPC): (Cont.)
**H01L 51/0054; H01L 51/0055; H01L 51/0068; H01L 51/0073; H01L 51/0094;** C07C 43/162; C07D 307/77; C08G 2261/124; C08G 2261/1412; C08G 2261/3162; C08G 2261/3246; C08G 2261/344; C08G 2261/512; C08G 2261/92; H01L 51/0004; H01L 51/0005; H01L 51/0558

C-Sets
**C08K 5/01, C08L 65/00;**
**C08K 5/45, C08L 65/00**

## Description

BACKGROUND OF THE INVENTION

1. Field of the Invention

The present invention relates to a composition for forming an organic semiconductor film, an organic semiconductor film and a method for manufacturing the same, an organic semiconductor element and a method for manufacturing the same, and an organic semiconductor compound. The invention is set out in the appended set of claims.

2. Description of the Related Art

An organic transistor having an organic semiconductor film (organic semiconductor layer) is used in a field effect transistor (FET) used in a liquid crystal display or an organic EL display, a Radio Frequency Identifier (RFID, RF tag), and the like, because the use of the organic transistor makes it possible to achieve lightening of weight and cost reduction and to achieve flexibilization.

JP 2009-267134 A relates to an organic transistor that has an organic semiconductor layer which includes at least one kind of compound represented by general formula 1.

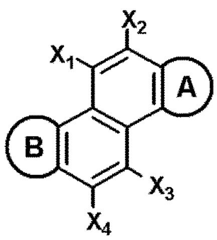

(1)

In the formula, $X_1$-$X_4$ independently represent a hydrogen atom, halogen atom, straight chain, branched, or annular alkyl group; straight chain, branched, or annular alkoxy group; straight chain, branched, or annular alkoxyalkyl group; or a substituted or an unsubstituted aryl group, where rings A, B represent a substituted or an unsubstituted thiophene ring or a substituted or an unsubstituted benzo [b] thiophene ring.

JP 5352260 B2 relates to an organic transistor having an organic semiconductor layer, the organic semiconductor layer contains at least one of a compound represented by general formula (1),

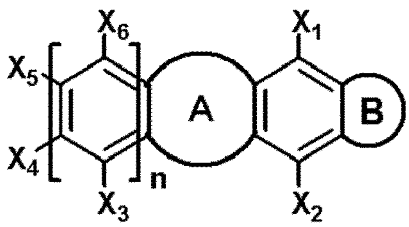

(1)

wherein $X_1$-$X_6$ each independently denote a hydrogen atom, a halogen atom, straight-chain, branched or cyclic alkyl, straight-chain, branched or cyclic alkoxy, straight-chain branched or cyclic alkoxyalkyl or substituted or unsubstituted aryl; a ring A denotes a thienothiophene ring; a ring B denotes a substituted or unsubstituted thiophene ring; and n denotes 0 or 1.

JP 2009-267140 A relates to an organic transistor having an organic semiconductor layer which includes at least one kind of compound represented by general formula 1

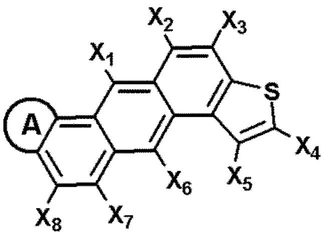

(1)

In the formula, $X_1$-$X_8$ independently represent a hydrogen atom, halogen atom, straight chain, branched, or annular alkyl group; a straight chain, branched, or annular alkoxy group; a straight chain, branched, or annular alkoxyalkyl group, or a substituted or an unsubstituted aryl group, where a ring A represents a substituted or an unsubstituted thiophene ring.

US 5,936,259 A1 relates to thin film transistors in which the active layer is a film of an organic semiconductor with a structure having two or three six-membered, fused aromatic rings with two five-membered, heterocyclic aromatic rings fused thereto. The five-membered rings are either substituted or unsubstituted. If substituted, the substituents are either alkyl or alkoxyalkyl with about two to about 18 carbon atoms.

JP 2010-177642 A relates to an organic transistor having an organic semiconductor layer, the organic semiconductor layer contains at least one of a compound represented by general formula (1),

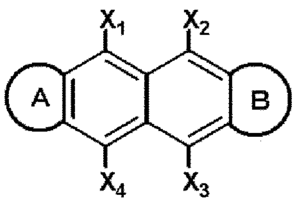

(1)

wherein $X_1$-$X_4$ each independently denote a hydrogen atom, a halogen atom, straight-chain, branched or cyclic alkyl, straight-chain, branched or cyclic alkoxy, straight-chain, branched or cyclic alkoxyalkyl or substituted or unsubstituted aryl; a ring A denotes a thienothiophene ring; a ring B denotes a substituted or unsubstituted thiophene ring, a substituted or unsubstituted benzo [b] thiophene ring or a substituted or unsubstituted thienothiophene ring.

As organic semiconductors of the related art, those described in JP2009-267132A and JP2012-510454A are known.

SUMMARY OF THE INVENTION

An object of the present invention is to provide a composition for forming an organic semiconductor film that has excellent preservation stability and makes the obtained organic semiconductor element exhibit excellent driving stability in the atmosphere. Another object of the present invention is to provide an organic semiconductor film using the composition for forming an organic semiconductor film, a method for manufacturing the organic semiconductor film, an organic semiconductor element, and a method for manufacturing the organic semiconductor element. Still other object of the present invention is to provide a novel organic semiconductor compound.

The aforementioned objects of the present invention were achieved by means described in the following <1> to <4>. Particular embodiments of the invention are set out in the dependent claims.

<1> A composition for forming an organic semiconductor film, comprising an organic semiconductor represented by the following Formula A-2 as a component A and a solvent as a component B, in which a content of a non-halogen-based solvent is equal to or greater than 50% by mass with respect to a total content of the component B, and a content of the component A is equal to or greater than 0.7% by mass and less than 15% by mass.

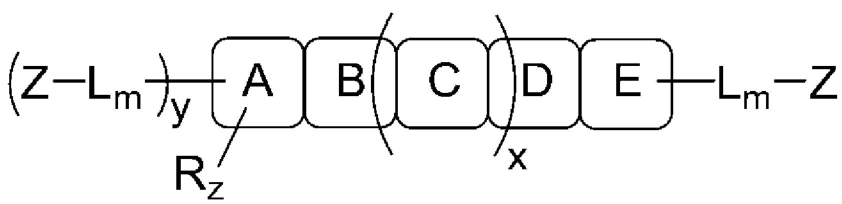

(A-2)

In Formula A-2, rings A to E each independently represent a benzene ring or a thiophene ring; R represents an alkyl group, an alkenyl group, an alkynyl group, an aromatic hydrocarbon group, an aromatic heterocyclic group, or a fluorine atom; L each independently represents a phenylene group or a thienylene group; Z each independently represents a group represented by Formula a-1; m each independently represents an integer of 1 to 4; when there are two or more L's, L's may be the same as or different from each other; when there are two or more Z's, Z's may be the same as or different from each other, x represents an integer of 1 to 3; y represents 0 or 1; z represents 0 or 1; and the symmetry of a ring-fused structure formed of the rings A to E is $C_2$, $C_{2v}$, or $C_{2h}$,

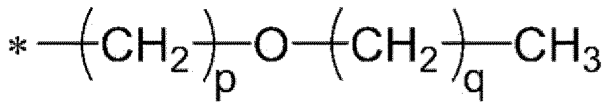

(a - 1)

In Formula a-1, p represents an integer of 1 to 20, q represents an integer of 0 to 20, and * represents a binding position with respect to other structures, wherein the rings A and E are thiophene rings and/or L is a thienylene group.

<2> A method for manufacturing an organic semiconductor film, comprising an application step of applying the composition for forming an organic semiconductor film described in <1> onto a substrate, and a drying step of removing a solvent from the applied composition.

<3> An organic semiconductor compound represented by Formula A-2.

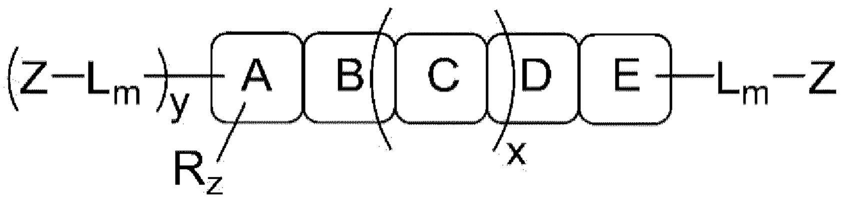

(A - 2)

In Formula A-2, rings A to E each independently represent a benzene ring or a thiophene ring; R represents an alkyl group, an alkenyl group, an alkynyl group, an aromatic hydrocarbon group, an aromatic heterocyclic group, or a fluorine atom; L each independently represents a phenylene group or a thienylene group; Z each independently represents a group represented by the following Formula a-1; m each independently represents an integer of 1 to 4; when there are two or more L's, L's may be the same as or different from each other; when there are two or more Z's, Z's may be the same as or different from each other; x represents an integer of 1 to 3; y represents 0 or 1; z represents 0 or 1; and the symmetry of a ring-fused structure formed of the rings A to E is $C_2$, $C_{2v}$, or $C_{2h}$.

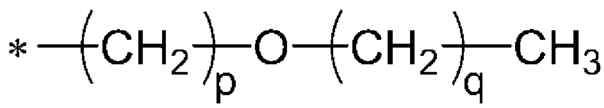

(a - 1)

In Formula a-1, p represents an integer of 1 to 20, q represents an integer of 0 to 20, and * represents a binding position with respect to other structures.

<4> An organic semiconductor film comprising the organic semiconductor compound according to <3>.

According to the present invention, it is possible to provide a composition for forming an organic semiconductor film that has excellent preservation stability and makes the obtained organic semiconductor element exhibit excellent driving stability in the atmosphere. Furthermore, according to the present invention, it is possible to provide an organic semiconductor film using the composition for forming an organic semiconductor film, a method for manufacturing the organic semiconductor film, an organic semiconductor element, and a method for manufacturing the organic semiconductor element. In addition, according to the present invention, it is possible to provide a novel organic semiconductor compound.

BRIEF DESCRIPTION OF THE DRAWINGS

Fig. 1 is a schematic cross-sectional view of an aspect of an organic semiconductor element of the present invention.
Fig. 2 is a schematic cross-sectional view of another aspect of the organic semiconductor element of the present

invention.

Fig. 3 is a plan view of a metal mask used in examples.

DESCRIPTION OF THE PREFERRED EMBODIMENTS

Hereinafter, the contents of the present invention will be specifically described. The constituents in the following description will be explained based on typical embodiments of the present invention, but the present invention is not limited to the embodiments. In the specification of the present application, "to" is used to mean that the numerical values listed before and after "to" are a lower limit and an upper limit respectively. Furthermore, in the present invention, an organic EL element refers to an organic electroluminescence element.

In the present specification, in a case where there is no description regarding whether a group (atomic group) is substituted or unsubstituted, the group includes both of a group having a substituent and a group not having a substituent. For example, an "alkyl group" includes not only an alkyl group not having a substituent (unsubstituted alkyl group) but also an alkyl group having a substituent (substituted alkyl group).

In the present specification, in some cases, a chemical structural formula is described as a simplified structural formula in which a hydrogen atom is omitted.

In the present invention, "mobility" refers to "carrier mobility" and means either of both of electron mobility and hole mobility.

In the present invention, "% by mass" and "% by weight" have the same definition, and "part by mass" and "part by weight" have the same definition.

In the present invention, a combination of preferred aspects is more preferable.

(Composition for forming organic semiconductor film and organic semiconductor compound)

A composition for forming an organic semiconductor film of the present invention contains an organic semiconductor represented by Formula A-2 as a component A and a solvent as a component B, in which a content of a non-halogen-based solvent is equal to or greater than 50% by mass with respect to a total content of the component B, and a content of the component A is equal to or greater than 0.7% by mass and less than 15% by mass.

As a result of repeating intensive investigation, the inventors of the present invention obtained knowledge that, by using a composition for forming an organic semiconductor film containing the aforementioned components A and B, excellent preservation stability of a composition for forming an organic semiconductor film can be obtained, and the obtained organic semiconductor film or organic semiconductor element exhibits high driving stability in the atmosphere. Based on the knowledge, the inventors accomplished the present invention.

The inventors of the present invention found that, if a halogen-based solvent is used as a solvent, the solubility of an organic semiconductor compound becomes excellent, but the obtained organic semiconductor film or organic semiconductor element exhibits low driving stability.

A detailed mechanism that brings about such effects is unclear. Presumably, because the component A has an alkoxyalkyl group (group represented by Z) on a terminal thereof, the solubility of the component A in a non-halogen-based solvent may be improved, and the preservation stability may become excellent. Furthermore, presumably, because the composition for forming an organic semiconductor film is a composition in which the use of a non-halogen-based solvent is suppressed, the driving stability of the obtained organic semiconductor film or organic semiconductor element in the atmosphere may be improved.

Hereinafter, each component used in the composition for forming an organic semiconductor film of the present invention will be described.

Component A:[0035] The component A is a compound represented by the following Formula A-2, and an organic semiconductor compound of the present invention is the compound represented by the following Formula A-2.

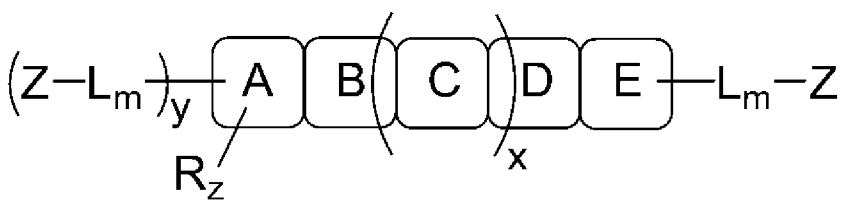

(A-2)

In Formula A-2, rings A to E each independently represent a benzene ring or a thiophene ring; R represents an alkyl group, an alkenyl group, an alkynyl group, an aromatic hydrocarbon group, an aromatic heterocyclic group, or a fluorine atom; L each independently represents a phenylene group or a thienylene group; Z each independently represents a

group represented by Formula a-1; m each independently represents an integer of 1 to 4; when there are two or more L's, L's may be the same as or different from each other; when there are two or more Z's, Z's may be the same as or different from each other; x represents an integer of 1 to 3; y represents 0 or 1; z represents 0 or 1; and the symmetry of a ring-fused structure formed of the rings A to E is $C_2$, $C_{2v}$, or $C_{2h}$.

In Formula A-2, the rings A to E each independently represent a benzene ring or a thiophene ring. It is preferable that 2 to 4 rings among the rings A to E are thiophene rings.

x represents an integer of 1 to 3. That is, the rings A to E have a ring-fused structure including 5 to 7 rings.
y represents 0 or 1, and is preferably 1.
z represents 0 or 1, and is preferably 0.

In Formula A-2, $L_m$-Z substitutes the ring E on a terminal of the fused polycyclic aromatic group constituted with the rings A to E. Furthermore, either or both of -$L_m$-Z and R substitute the ring A present on the other terminal. In the compound represented by Formula A-2, when y is 1, z is preferably 0, and when y is 0, z is preferably 1.

In Formula A-2, R represents an alkyl group, an alkenyl group, an alkynyl group, an aromatic hydrocarbon group, an aromatic heterocyclic group, or a fluorine atom. The alkyl group may be linear, branched, or cyclic, and is preferably linear. The alkyl group has 1 to 20 carbon atoms, preferably has 1 to 12 carbon atoms, and more preferably 1 to 8 carbon atoms. The alkenyl group has 2 to 20 carbon atoms, preferably 2 to 12 carbon atoms, and more preferably 2 to 8 carbon atoms. The alkynyl group has 2 to 20 carbon atoms, preferably has 2 to 12 carbon atoms, and more preferably has 2 to 8 carbon atoms. The alkenyl group and the alkynyl group may be linear, branched, or cyclic, and are preferably linear. The aromatic hydrocarbon group has 6 to 30 carbon atoms, preferably has 6 to 20 carbon atoms, and more preferably has 6 to 10 carbon atoms. The aromatic hydrocarbon group is particularly preferably a phenyl group. The aromatic heterocyclic group preferably has at least one heteroatom selected from the group consisting of a sulfur atom, an oxygen atom, a nitrogen atom, and a selenium atom, and more preferably has a heteroatom selected from the group consisting of a sulfur atom, a nitrogen atom, and an oxygen atom. The heterocyclic group may be monocyclic or polycyclic, and is a 5- to 30-membered ring, preferably 5- to 20-membered ring, and more preferably a 5- to 10-membered ring.

Among these, R is preferably an alkyl group, and particularly preferably a linear alkyl group.

In the compound represented by Formula A-2, the rings A and E are thiophene rings and/or L is a thienylene ring and m is an integer of 1 to 4. That is, the group represented by Formula a-1 is substituted with a thiophene ring. Furthermore, in the group represented by Formula a-1, p is preferably an integer of 1 to 6.

In Formula A-2, the symmetry of the ring-fused structure formed of the rings A to E is $C_2$, $C_{2v}$, or $C_{2h}$. If the symmetry is $C_2$, $C_{2v}$, or $C_{2h}$, a well-ordered crystal structure is easily obtained, and high mobility is easily exhibited.

Regarding the symmetry of a ring-fused structure, the description of "Molecular Symmetry and Theory of Groups" (Masao Nagazaki, Tokyo Kagaku Dojin) can be referred to.

Examples of the component A and the organic semiconductor compound of the present invention (compound represented by Formula A-2) will be shown below, but the present invention is not limited to the examples.

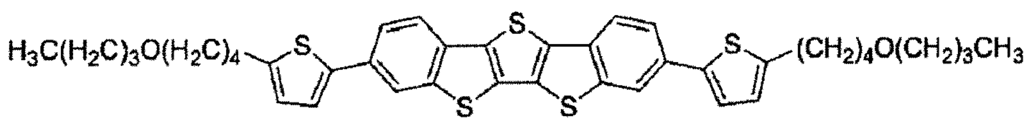

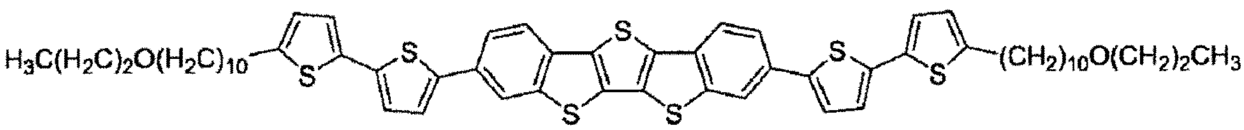

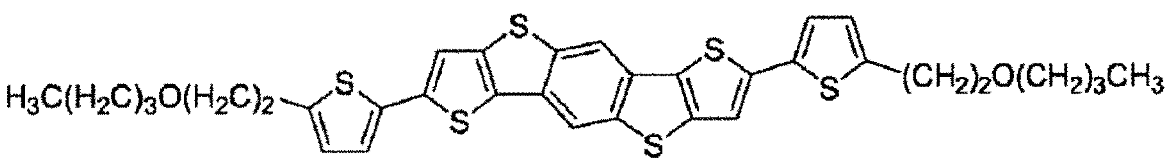

A molecular weight of the component A is not particularly limited, but is preferably equal to or less than 1,500, more preferably equal to or less than 1,000, and even more preferably equal to or less than 800. If the molecular weight is equal to or less than the aforementioned upper limit, the solubility of the component A in a solvent can be improved.

In contrast, from the viewpoint of the qualitative stability of a thin film, the molecular weight is preferably equal to or greater than 400, more preferably equal to or greater than 450, and even more preferably equal to or greater than 500.

One kind of component A may be used singly, or two or more kinds thereof may be used in combination.

A method for manufacturing the component A is not particularly limietd, and the component A can be synthesized with reference to known methods. Specifically, it is possible to refer to JP2013-191821A, JP2009-246140A, JP2011-32268A, JP2009-54810A, JP2011-526588A, JP2012-510454A, JP2010-520241A, JP2010-6794A, JP2006-176491A, US2008/0142792A, WO2010/098372A, Adv. Mater. 2013, 25, 6392., Chem. Commun. 2014, 50, 5342., Appl. Phys. Express, 2013, 6, 076503., and Scientific Reports 2014, 4, 5048.

A content of the component A in the composition for forming an organic semiconductor film of the present invention is, with respect to a total amount of solid contents, preferably 30% to 100% by mass, more preferably 50% to 100% by mass, and even more preferably 70% to 100% by mass. In a case where the composition does not contain a binder polymer which will be described later, the aforementioned total content is preferably 90% to 100% by mass, and more preferably 95% to 100% by mass.

The content of the component A in the composition for forming an organic semiconductor film of the present invention is equal to or greater than 0.7% by mass and less than 15% by mass. If the content of the component A is less than 0.7% by mass, a concentration of the component A in the composition for forming an organic semiconductor film is low, and it is difficult to obtain an organic semiconductor film and an organic semiconductor element having high mobility and driving stability. In contrast, if the content of the component A is equal to or greater than 15% by mass, the concentration of the component A is high, and the preservation stability deteriorates.

The content of the component A in the composition for forming an organic semiconductor film is preferably 1.0% to 10% by mass, more preferably 1.25% to 10% by mass, and even more preferably 1.5% to 10% by mass.

Component B: solvent

The composition for forming an organic semiconductor film of the present invention contains a solvent as a component B, and a content of a non-halogen-based solvent is equal to or greater than 50% by mass and less than 100% by mass with respect to a total content of the component B. Herein, the "non-halogen-based solvent" is a solvent not having a halogen atom.

If the content of the non-halogen-based solvent is less than 50% by mass, an organic semiconductor film and an organic semiconductor element having excellent driving stability cannot be obtained.

The component B is not particularly limited as long as the component B dissolves the component A such that a solution having a desired concentration can be prepared or the component B can disperse the component A.

Examples of the non-halogen-based solvent include an aliphatic hydrocarbon-based organic solvent such as pentane, hexane, heptane, octane, decane, dodecane, isopentane, isohexane, isooctane, cyclohexane, methylcyclohexane, cyclopentane, or decalin; an aromatic hydrocarbon-based solvent such as benzene, toluene, o-xylene, m-xylene, p-xylene, ethylbenzene, mesitylene, tetralin, cyclohexylbenzene, diethylbenzene, or 1-methylnaphthalene; an ester-based solvent such as methyl formate, ethyl formate, propyl formate, methyl acetate, ethyl acetate, isopropyl acetate, n-propyl acetate, isobutyl acetaet, n-butyl acetate, amyl acetate, methyl propionate, or ethyl propionate, an alcohol-based solvent such as methanol, ethanol, propanol, butanol, pentanol, hexanol, cyclohexanol, $\alpha$-terpineol, methyl cellosolve, ethyl cellosolve, or ethylene glycol, a ketone-based solvent such as acetone, methyl ethyl ketone, methyl isobutyl ketone, cyclohexanone, 2-hexanone, 2-heptanone, or 2-octanone; an alkylene glycol-based solvent such as diethylene glycol ethyl ether, diethylene glycol diethyl ether, propylene glycol monomethyl ehter, propylene glycol monoethyl ether, propylene glycol monopropyl ether, propylene glycol monobutyl ether, propylene glycol monomethyl ether acetate, diethylene glycol methyl ether acetate, diethylene glycol ethyl ether acetate, diethylene glycol propyl ether acetate, diethylene glycol isopropyl ether acetate, diethylene glycol butyl ether acetate, diethylene glycol-t-butyl ether acetate, triethylene glycol methyl ether acetate, triethylene glycol ethyl ether acetate, triethylene glycol propyl ether acetate, triethylene glycol isopropyl ether acetate, triethylene glycol butyl ether acetate, triethylene glycol-t-butyl ether acetate, dipropylene glycol dimethyl ether, or dipropylene glycol monobutyl ether; an ether-based solvent such as diethyl ether, dipropyl ether, diisopropyl ether, dibutyl ether, dihexyl ether, ethyl vinyl ether, butyl vinyl ether, anisole, butyl phenyl ether, pentyl phenyl ether, methoxytoluene, benzyl ethyl ether, diphenyl ether, dibenzyl ether, dioxane, furan, or tetrahydrofuran; an amide·imide-based solvent such as N,N-dimethylformamide, N,N-dimethylacetamide, 1-methyl-2-pyrrolidone, or 1-methyl-2-imidazolidinone; a sulfoxide-based solvent such as dimethyl sulfoxide; a nitrile-based solvent such as acetonitrile; or the like, but the non-halogen-based solvent is not particularly limited.

Examples of halogen-based solvents which may be used in combination with the above non-halogen-based solvents include dichloromethane, chloroform, tetrachloromethane, dichloroethane, trichloroethane, chlorobenzene, chlorotoluene, 1,2-dichlorobenzene, trichlorobenzene (1,2,4-trichlorobenzene or the like), and the like.

From the viewpoint of the stability of the composition for forming an organic semiconductor film and from the viewpoint of forming a uniform film, a boiling point of the component B under normal pressure is preferably equal to or

higher than 100°C, more preferably equal to or higher than 150°C, even more preferably equal to or higher than 175°C, and particularly preferably equal to or higher than 200°C. From the viewpoint of drying the component B after the application of the composition for forming an organic semiconductor film, the boiling point of the component B is preferably equal to or lower than 300°C, more preferably equal to or lower than 250°C, and even more preferably equal to or lower than 220°C.

The non-halogen-based solvent contains an aromatic solvent preferably in an amount of equal to or greater than 50% by mass. If the non-halogen-based solvent contains the aromatic solvent in an amount of equal to or greater than 50% by mass, the solubility of the component A becomes excellent, and an organic semiconductor film or an organic semiconductor element having high driving stability is obtained.

The non-halogen-based solvent contains the aromatic solvent more preferably in an amount of equal to or greater than 70% by mass, and even more preferably in an amount of equal to or greater than 85% by mass. It is particularly preferable that the non-halogen-based solvent is totally composed of the aromatic solvent, that is, the aromatic solvent accounts for 100% by mass of the non-halogen-based solvent.

As the component B, a non-halogen-based aromatic solvent having a boiling point of equal to or higher than 100°C is preferable. Specific examples thereof include toluene (boiling point: 111°C), xylene (boiling point: 138°C to 144°C), anisole (boiling point: 154°C), mesitylene (boiling point: 165°C), diethyl benzene (boiling point: 180°C to 182°C), and tetralin (boiling point: 208°C).

One kind of component B may be used singly, or two or more kinds thereof may be used in combination.

The component B should be appropriately added such that the content of the component A in the composition for forming an organic semiconductor film and an amount of total solid contents thereof which will be described later fall into a desired range.

Component C: binder polymer

The composition for forming an organic semiconductor film of the present invention preferably contains a binder polymer as a component C.

Furthermore, an organic semiconductor film and an organic semiconductor element of the present invention may be an organic semiconductor element having a layer containing the aforementioned organic semiconductor compound and a layer containing the binder polymer.

The type of binder polymer is not particularly limited, and known binder polymers can be used.

Examples of the binder polymer include an insulating polymer such as polystyrene, polycarbonate, polyarylate, polyester, polyamide, polyimide, polyurethane, polysiloxane, polysulfone, polymethyl methacrylate, polymethyl acrylate, cellulose, polyethylene, or polypropylene and a copolymer of these, a semiconductor polymer such as polysilane, polycarbazole, polyarylamine, polyfluorene, polythiophene, polypyrrole, polyaniline, polyparaphenylene vinylene, polyacene, or polyheteroacene and a copolyme of these, rubber, and thermoplastic elastomer.

Among these, as the binder polymer, a benzene ring-containing polymer compound (polymer having a benzene ring group-containing monomer unit) is preferable. A content of the benzene ring group-containing monomer unit is not particularly limited, but is preferably equal to or greater than 50 mol%, more preferably equal to or greater than 70 mol%, and even more preferably equal to or greater than 90 mol% with respect to all of the monomer units. An upper limit of the content is not particularly limited and is, for example, 100 mol%.

Examples of the aforementioned binder polymer include polystyrene, poly($\alpha$-methylstyrene), polyvinyl cinnamate, poly(4-vinylphenyl), poly(4-methylstyrene), poly[bis(4-phenyl)(2,4,6-trimethylphenyl)amine], poly[2,6-(4,4-bis(2-ethylhexyl)-4H cyclopenta[2,1-b;3,4-b']dithiophene)-alt-4,7-(2,1,3-benzothiadiazole)], and the like. Among these, poly($\alpha$-methylstyrene) is particularly preferable.

A weight-average molecular weight of the binder polymer is not particularly limited, but is preferably 1,000 to 2,000,000, more preferably 3,000 to 1,000,000, and even more preferably 5,000 to 600,000.

It is preferable that, in the component B, the solubility of the binder polymer is higher than the solubility of the component A. If this aspect is adopted, the mobility and heat stability of the obtained organic semiconductor are further improved.

A content of the binder polymer in the composition for forming an organic semiconductor film of the present invention is, with respect to 100 parts by mass of the content of the component A, preferably 1 to 10,000 parts by mass, more preferably 10 to 1,000 parts by mass, even more preferably 25 to 400 parts by mass, and most preferably 50 to 200 parts by mass. If the content is within the above range, the mobility and film uniformity of the obtained organic semiconductor are further improved.

<Other components>

The composition for forming an organic semiconductor film of the present invention may contain other components in addition to the components A to C.

As other components, known additives and the like can be used.

A concentration of total solid cotnents in the composition for forming an organic semiconductor film of the present invention is preferably equal to or higher than 1.5% by mass. Herein, the "solid contents" is an amount of components excluding a volatile component such as a solvent. That is, the concentration of total solid contents including the components A and C is preferably equal to or higher than 1.5% by mass. It is preferable that the concentration of solid contents is equal to or higher than 1.5% by mass, because then excellent film formability is exhibited in various printing methods.

The concentration of total solid contents in the composition for forming an organic semiconductor film is more preferably equal to or higher than 2% by mass, and even more preferably equal to or higher than 3% by mass. An upper limit of the concentration is not limited. From the viewpoint of the solubility of the component A or the like, the upper limit is preferably equal to or lower than 20% by mass, more preferably equal to or lower than 15% by mass, and even more preferably equal to or lower than 10% by mass. If the upper limit is within the above range, the preservation stability and film formability become excellent, and the mobility of the obtained organic semiconductor is further improved.

The composition for forming an organic semiconductor film of the present invention is suitable for ink jet printing and/or flexographic printing.

A viscosity of the composition for forming an organic semiconductor film of the present invention is not particularly limited. From the viewpoint of further improving suitability for various printing methods, particularly, suitability for ink jet printing and flexographic printing, the viscosity is preferably 3 to 100 mPa·s, more preferably 5 to 50 mPa·s, even more preferably 5 to 40 mPa·s, and particularly preferably 9 to 40 mPa·s. The viscosity in the present invention is a viscosity at 25°C.

As a method for measuring the viscosity, a method based on JIS Z8803 is preferable.

A method for manufacturing the composition for forming an organic semiconductor film of the present invention is not particularly limited, and known methods can be adopted. For example, by adding a predetermined amount of component A to the component B and appropriately stirring the mixture, a desired composition can be obtained. In a case where the component C is used, the composition can be suitably prepared by simultaneously or sequentially adding the components A and C.

(Organic semiconductor film and organic semiconductor element)

The organic semiconductor film of the present invention is manufactured using the composition for forming an organic semiconductor film of the present invention, and the organic semiconductor element of the present invention is manufactured using the composition for forming an organic semiconductor film of the present invention.

A method for manufacturing the organic semiconductor film or the organic semiconductor element by using the composition for forming an organic semiconductor film of the present invention is not particularly limited, and known methods can be adopted. Examples thereof include a method for manufacturing an organic semiconductor film or an organic semiconductor element by applying the composition onto a predetermined substrate and performing a drying treatment if necessary.

A method for applying the composition onto a substrate is not particularly limited, and known methods can be adopted. Examples thereof include an ink jet printing method, a flexographic printing method, a bar coating method, a spin coating method, a knife coating method, a doctor blade method, a drop casting method, and the like. Among these, an ink jet printing method, a flexographic printing method, a spin coating method, and a drop casting method are preferable, and an ink jet printing method and a flexographic printing method are particularly preferable.

As the flexographic printing method, an aspect in which a photosensitive resin plate is used as a flexographic printing plate is suitably exemplified. By printing the composition onto a substrate according to the aspect, a pattern can be easily formed.

Among these, the method for manufacturing an organic semiconductor film of the present invention and the method for manufacturing an organic semiconductor element of the present invention more preferably include an application step of applying the composition for forming an organic semiconductor film of the present invention onto a substrate, and a removing step of removing a solvent from the applied composition.

The drying treatment in the removing step is a treatment performed if necessary, and the optimal treatment conditions are appropriately selected according to the type of the specific compound and the solvent used. In view of further improving the mobility and heat stability of the obtained organic semiconductor and improving productivity, a heating temperature is preferably 30°C to 150°C and more preferably 40°C to 100°C, and a heating time is preferably 1 to 300 minutes and more preferably 10 to 120 minutes.

A film thickenss of the formed organic semiconductor film of the present invention is not particularly limited. From the viewpoint of the mobility and heat stability of the obtained organic semiconductor, the film thickness is preferably 5 to 500 nm and more preferably 20 to 200 nm.

The organic semiconductor film of the present invention can be suitably used in an organic semiconductor element, and can be particularly suitably used in an organic transistor (organic thin film transistor).
The organic semiconductor film of the present invention is suitably prepared using the composition for forming an organic semiconductor film of the present invention.

<Organic semiconductor element>

The organic semiconductor element is not particularly limited, but is preferably an organic semiconductor element having 2 to 5 terminals, and even more preferably an organic semiconductor element having 2 or 3 terminals.
Furthermore, the organic semiconductor element is preferably an element which does not use a photoelectric function.
In addition, the organic semiconductor element of the present invention is preferably a non-light emitting organic semiconductor element.
Examples of the 2-terminal element include a rectifier diode, a constant voltage diode, a PIN diode, a Schottky barrier diode, a surge protection diode, a diac, a varistor, a tunnel diode, and the like.
Examples of the 3-terminal element include a bipolar transistor, a Darlington transistor, a field effect transistor, insulated gate bipolar transistor, a uni-junction transistor, a static induction transistor, a gate turn thyristor, a triac, a static induction thyristor, and the like.
Among these, a rectifier diode and transistors are preferable, and a field effect transistor is more preferable. Examples of the field effect transistor preferably include an organic thin film transistor.
An aspect of the organic thin film transistor of the present invention will be described with reference to a drawing.
Fig. 1 is a schematic cross-sectional view of an aspect of an organic semiconductor element (organic thin film transistor (TFT)) of the present invention.
In Fig. 1, an organic thin film transistor 100 includes a substrate 10, a gate electrode 20 disposed on the substrate 10, a gate insulating film 30 covering the gate electrode 20, a source electrode 40 and a drain electrode 42 which contact a surface of the gate insulating film 30 that is on the side opposite to the gate electrode 20 side, an organic semiconductor film 50 covering a surface of the gate insulating film 30 between the source electrode 40 and the drain electrode 42, and a sealing layer 60 covering each member. The organic thin film transistor 100 is a bottom gate-bottom contact type organic thin film transistor.
In Fig. 1, the organic semiconductor film 50 corresponds to a film formed of the composition described above.
Hereinafter, the substrate, the gate electrode, the gate insulating film, the source electrode, the drain electrode, the organic semiconductor film, the sealing layer, and methods for forming each of these will be specifically described.

[Substrate]

The substrate plays a role of supporting the gate electrode, the source electrode, the drain electrode, and the like which will be described later.
The type of the substrate is not particularly limited, and examples thereof include a plastic substrate, a glass substrate, a ceramic substrate, and the like. Among these, from the viewpoint of applicability to each device and costs, a glass substrate or a plastic substrate is preferable.
Examples of materials of the plastic substrate include a thermosetting resin (for example, an epoxy resin, a phenol resin, a polyimide resin, or a polyester resin (for example, polyethylene terephthalate (PET) or polyethylene naphthalate (PEN)) and a thermoplastic resin (for example, a phenoxy resin, a polyethersulfone, polysulfone, or polyphenylene sulfone).
Examples of materials of the ceramic substrate include alumina, aluminum nitride, zirconia, silicon, silicon nitride, silicon carbide, and the like.
Examples of materials of the glass substrate include soda lime glass, potash glass, borosilicate glass, quartz glass, aluminosilicate glass, lead glass, and the like.

[Gate electrode, source electrode, and drain electrode]

Examples of materials of the gate electrode, the source electrode, and the drain electrode include a metal such as gold (Au), silver, aluminum (Al), copper, chromium, nickel, cobalt, titanium, platinum, tantalum, magnesium, calcium, barium, or sodium; a conductive oxide such as $InO_2$, $SnO_2$, or indium tin oxide (ITO); a conductive polymer such as polyaniline, polypyrrole, polythiophene, polyacetylene, or polydiacetylene; a semiconductor such as silicon, germanium, or gallium arsenide; a carbon material such as fullerene, carbon nanotubes, or graphite; and the like. Among these, a metal is preferable, and silver and aluminum are more preferable.
A thickness of each of the gate electrode, the source electrode, and the drain electrode is not particularly limited,

but is preferably 20 to 200 nm.

A method for forming the gate electrode, the source electrode, and the drain electrode is not particularly limited, but examples thereof include a method of vacuum vapor-depositing or sputtering an electrode material onto a substrate, a method of coating a substrate with a composition for forming an electrode, a method of printing a composition for forming an electrode onto a substrate, and the like. Furthermore, in a case where the electrode is patterned, examples of the patterning method include a photolithography method; a printing method such as ink jet printing, screen printing, offset printing, or relief printing; a mask vapor deposition method; and the like.

[Gate insulating film]

Examples of materials of the gate insulating film include a polymer such as polymethyl methacrylate, polystyrene, polyvinylphenol, polyimide, polycarbonate, polyester, polyvinylalcohol, polyvinyl acetate, polyurethane, polysulfone, polybenzoxazole, polysilsesquioxane, an epoxy resin, or a phenol resin; an oxide such as silicon dioxide, aluminum oxide, or titanium oxide; a nitride such as silicon nitride; and the like. Among these materials, in view of the compatibility with the organic semiconductor film, a polymer is preferable.

In a case where a polymer is used as the material of the gate insulating film, it is preferable to use a cross-linking agent (for example, melamine) in combination. If the cross-linking agent is used in combination, the polymer is cross-linked, and durability of the formed gate insulating film is improved.

A film thickness of the gate insulating film is not particularly limited, but is preferably 100 to 1,000 nm.

A method for forming the gate insulating film is not particularly limited, but examples thereof include a method of coating a substrate, on which the gate electrode is formed, with a composition for forming a gate insulating film, a method of vapor-depositing or sputtering the material of the gate insulating film onto a substrate on which the gate electrode is formed, and the like. A method for coating the aforementioned substrate with the composition for forming a gate insulating film is not particularly limited, and it is possible to use a known method (a bar coating method, a spin coating method, a knife coating method, or a doctor blade method).

In a case where the gate insulating film is formed by coating the substrate with the composition for forming a gate insulating film, for the purpose of removing the solvent, causing cross-linking, or the like, the composition may be heated (baked) after coating.

[Binder polymer layer]

The organic semiconductor element of the present invention preferably has the aforementioned binder polymer layer between the aforementioned organic semiconductor layer and the insulating film, and more preferably has the polymer layer between the aforementioned organic semiconductor layer and the gate insulating film. A film thickness of the binder polymer layer is not particularly limited, but is preferably 20 to 500 nm. The binder polymer layer should be a layer containing the aforementioned polymer, and is preferably a layer composed of the aforementioned binder polymer.

A method for forming the binder polymer layer is not particularly limited, and known methods (a bar coating method, a spin coating method, a knife coating method, a doctor blade method, and an ink jet method) can be used.

In a case where the binder polymer layer is formed by performing coating by using a composition for forming a binder polymer layer, for the purpose of removing a solvent or causing cross-linking, or the like, the composition may be heated (baked) after coating.

[Sealing layer]

From the viewpoint of durability, the organic semiconductor element of the present invention preferably includes a sealing layer as an outermost layer. In the sealing layer, a known sealant can be used.

A thickness of the sealing layer is not particularly limited, but is preferably 0.2 to 10 μm.

A method for forming the sealing layer is not particularly limited, but examples thereof include a method of coating a substrate, on which the gate electrode, the gate insulating film, the source electrode, the drain electrode, and the organic semiconductor film are formed, with a composition for forming a sealing layer, and the like. Specific examples of the method of coating the substrate with the composition for forming a sealing layer are the same as the examples of the method of coating the substrate with the composition for forming a gate insulating film. In a case where the organic semiconductor film is formed by coating the substrate with the composition for forming a sealing layer, for the purpose of removing the solvent, causing cross-linking, or the like, the composition may be heated (baked) after coating.

Fig. 2 is a schematic cross-sectional view of another aspect of the organic semiconductor element (organic thin film transistor) of the present invention.

In Fig. 2, an organic thin film transistor 200 includes the substrate 10, the gate electrode 20 disposed on the substrate 10, the gate insulating film 30 covering the gate electrode 20, the organic semiconductor film 50 disposed on

the gate insulating film 30, the source electrode 40 and the drain electrode 42 disposed on the organic semiconductor film 50, and the sealing layer 60 covering each member. Herein, the source electrode 40 and the drain electrode 42 are formed using the aforementioned composition of the present invention. The organic thin film transistor 200 is a top contact-type organic thin film transistor.

The substrate, the gate electrode, the gate insulating film, the source electrode, the drain electrode, the organic semiconductor film, and the sealing layer are as described above.

In Figs. 1 and 2, the aspects of the bottom gate-bottom contact type organic thin film transistor and the bottom gate-top contact type organic thin film transistor were specifically described. However, the organic semiconductor element of the present invention can also be suitably used in a top gate-bottom contact type organic thin film transistor and a top gate-top contact type organic thin film transistor.

The aforementioned organic thin film transistor can be suitably used in electronic paper, a display device, and the like.

Examples

Hereinafter, the present invention will be more specifically described based on examples. The materials and the amount thereof used, the proportion of the materials, the content and procedure of treatments, and the like described in the following examples can be appropriately changed within a scope that does not depart from the gist of the present invention. Accordingly, the scope of the present invention is not limited to the following specific examples. Herein, unless otherwise specified, "part" and "%" are based on mass.

(Organic semiconductor)

The structures of compounds 1 to 19 and comparative compounds 1 to 8 used in organic semiconductor layers will be shown below.

The compound 1 was synthesized with reference to the method described in JP2013-191821A.

The compound 2 was synthesized with reference to the method described in JP2009-246140A.

The compounds 3 to 5 were synthesized with reference to the method described in JP2011-32268A.

The compounds 6 to 10 were synthesized with reference to the methods described in JP2009-54810A, JP2011-526588A, and JP2012-510454A.

The compound 11 was synthesized with reference to the method described in JP2010-520241A.

The compound 12 was synthesized with reference to the method described in Adv. Mater. 2013, 25, 6392.

The compound 13 was synthesized with reference to the method described in Chem. Commun. 2014, 50, 5342.

The compound 14 was synthesized with reference to the method described in US2008/0142792A.

The compound 15 was synthesized with reference to the method described in WO2010/098372A.

The compound 16 was synthesized with reference to the method described in Appl. Phys. Express 2013, 6, 076503.

The compound 17 was synthesized with reference to the method described in Scientific Reports 2014, 4, 5048.

The compound 18 was synthesized with reference to the method described in JP2010-6794A.

The compound 19 was synthesized with reference to the method described in JP2006-176491A.

The comparative compounds 1 and 2 are examples compounds 27 and 56 of JP2009-267132A respectively.

The comparative compounds 3 and 4 are compounds used in Examples 1 and 2 of JP2012-510454A.

The comparative compounds 5 and 6 are compounds 41 and 7 described in JP2011-32268A.

The comparative compound 7 is a compound (12) described in WO2010/098372A.

The comparative compound 8 was synthesized with reference to the methods described in JP2009-54810A and JP2011-526588A.

Through high-performance liquid chromatography (manufactured by TOSOH CORPORATION, TSKgel ODS-100Z), it was confirmed that all of the compounds had a purity (area ratio for absorption intensity at 254 nm) of equal to or higher than 99.8%. The structures of the compounds were identified by $^1$H-NMR.

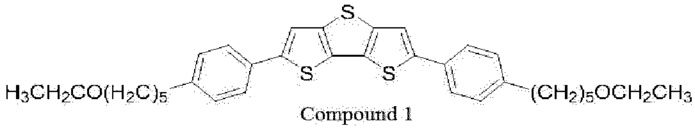

Compound 1

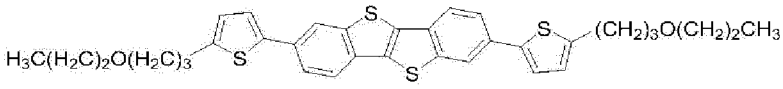

Compound 2

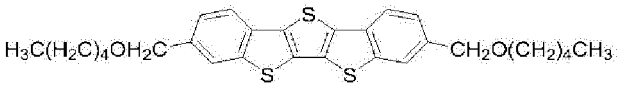

Compound 3

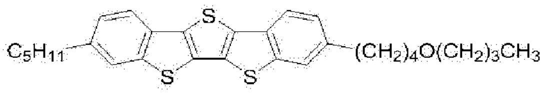

Compound 4

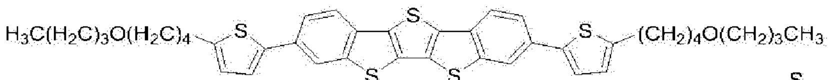

Compound 5

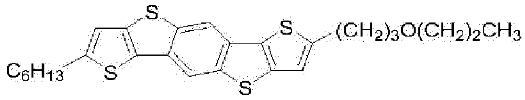

Compound 6

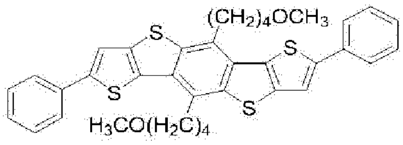

Compound 7

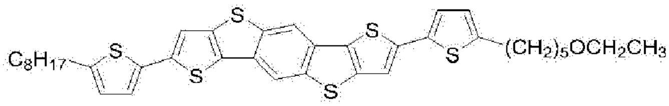

Compound 8

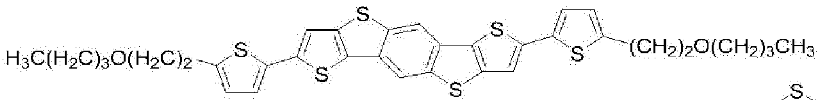

Compound 9

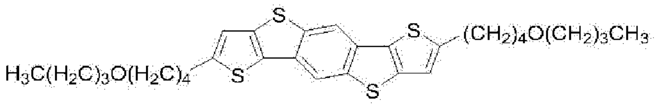

Compound 10

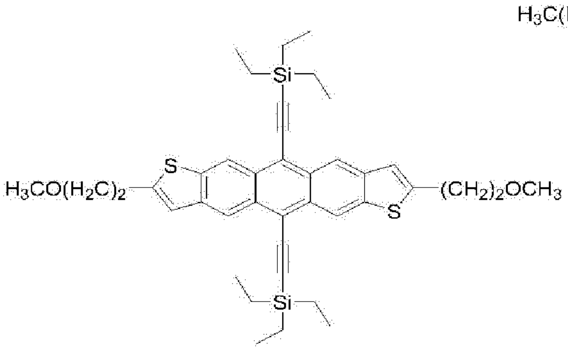

Compound 11

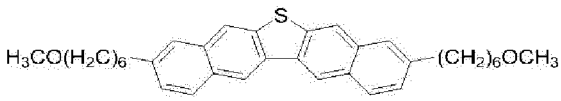

Compound 12

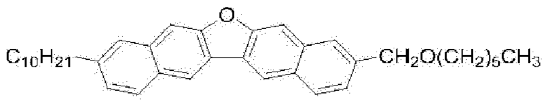

Compound 13

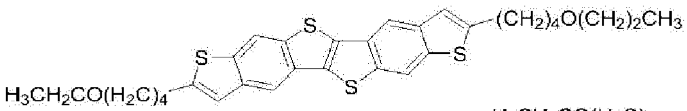

Compound 14

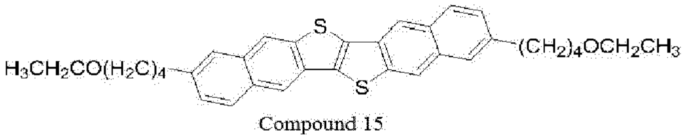

Compound 15

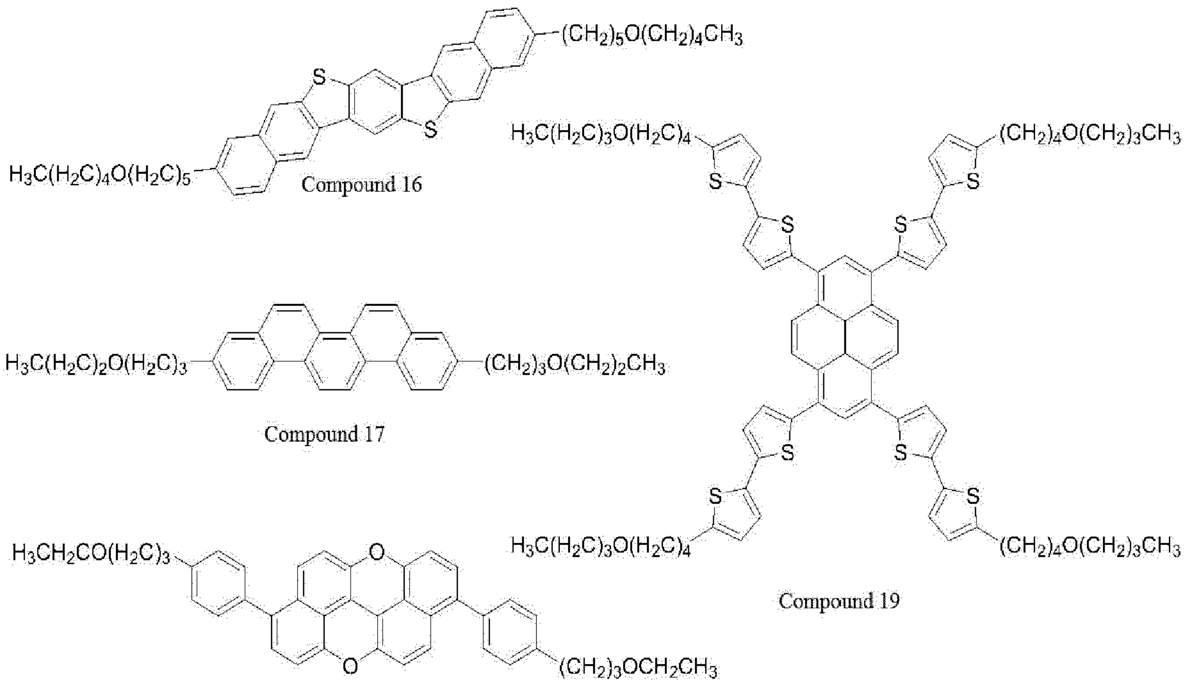

Compound 16

Compound 17

Compound 19

Compound 18

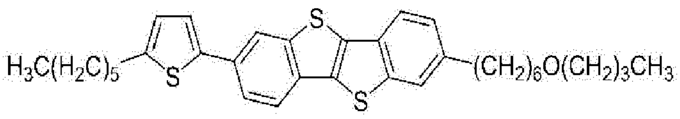

Comparative compound 1

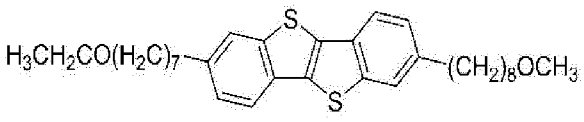

Comparative compound 2

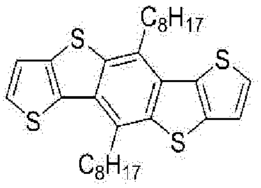

Comparative compound 3

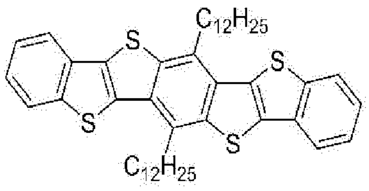

Comparative compound 4

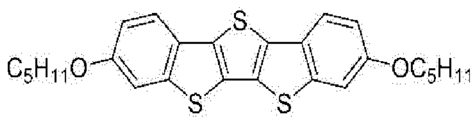

Comparative compound 5

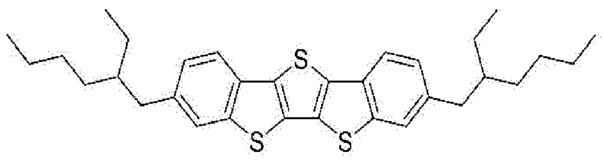

Comparative compound 6

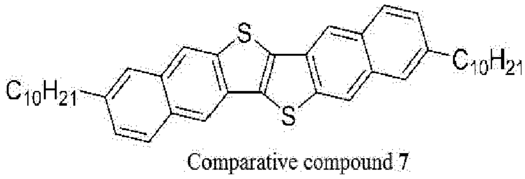

Comparative compound 7

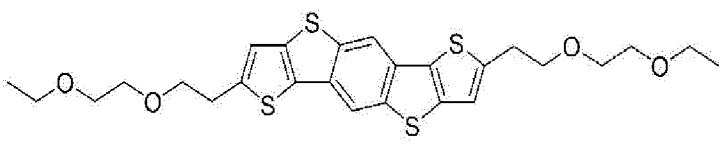

Comparative compound 8

(Solvent)

The solvents used in examples and comparative examples will be shown below.

- Tetralin: boiling point 208°C, manufactured by Sigma-Aldrich Co. LLC.
- Mesitylene: boiling point 165°C, manufactured by Sigma-Aldrich Co. LLC.
- Cyclohexanone: boiling point 156°C, manufactured by Sigma-Aldrich Co. LLC.
- Diethylbenzene (isomer mixture): boiling point 180°C to 182°C, manufactured by TOKYO CHEMICAL INDUSTRY CO., LTD.
- Anisole: boiling point 154°C, manufactured by Sigma-Aldrich Co. LLC.
- N-methylpyrrolidone: boiling point 202°C, manufactured by Sigma-Aldrich Co. LLC.
- Chlorobenzene: boiling point 131°C, manufactured by Sigma-Aldrich Co. LLC.
- Chloroform: boiling point 61°C, manufactured by Sigma-Aldrich Co. LLC.

(Binder polymer)

The binder polymers used in examples and comparative examples will be shown below.

- PαMS: poly-α-methylsytrene, weight-average molecular weight 437,000, manufactured by Sigma-Aldrich Co. LLC.
- PTAA: poly[bis(4-phenyl)(2,4,6-trimethylphenyl)amine], number-average molecular weight 7,000 to 10,000, manufactured by Sigma-Aldrich Co. LLC.
- PCPDTBT: poly[2,6-(4,4-bis(2-ethylhexyl)-4H cyclopenta[2,1-b;3,4-b']dithiophene)-alt-4,7-(2,1,3-benzothiadiazole)], weight-average molecular weight 7,000 to 20,000, manufactured by Sigma-Aldrich Co. LLC.

<Preparation of composition for forming organic semiconductor film>

The organic semiconductor (compound)/binder polymer/solvent described in Table 1 were weighed out in a glass vial at a concentration described in Table 1 and stirred and mixed together for 10 minutes by using MIX ROTOR (manufactured by AS ONE Corporation). The mixture was filtered through a 0.5 μm membrane filter, thereby obtaining a coating solution for forming an organic semiconductor film. The mark "-" in the column of binder polymer in the table means that the binder polymer was not added.

The concentration of the organic semiconductor and the binder polymer is a concentration (% by mass) with respect to a total amount of the composition for forming an organic semiconductor film.

<Preparation of TFT element>

Onto a glass substrate (EAGLE XG: manufactured by Corning Incorporated), Al to be a gate electrode was vapor-deposited (thickness: 50 nm). The AL was spin-coated with a composition for forming a gate insulatnig film (PGMEA (propylene glycol monomethyl ether acetate) solution (concentration of solid contents: 2% by mass) of polyvinylphenol/melamine = 1 part by mass/1 part by mass (w/w)), followed by baking for 60 minutes at 150°C, thereby forming a gate insulating film having a film thickness of 400 nm. On the gate insulating film, by using a silver ink (H-1, manufactured by Mitsuibishi Materials Corporation) and an ink jet device DMP-2831 (manufactured by FUJIFILM Dimatix, Inc.), patterns of a source electrode and a drain electrode (channel length: 40 μm, channel width: 200 μm) were drawn. The substrate was then sintered by being baked for 30 minutes at 180°C in an oven such that a source electrode and a drain electrode were formed, thereby obtaining an element substrate for TFT characteristic evaluation.

The element substrate for TFT characteristic evaluation was spin-coated with each composition for forming an organic semiconductor film (for 10 seconds at 500 rpm and then for 30 seconds at 1,000 rpm) and then dried for 10 minutes at 100°C on a hot plate such that an organic semiconductor layer was formed, thereby obtaining a bottom gate-bottom contact type organic TFT element.

Furthermore, the composition for forming an organic semiconductor film was applied to the substrate by ink jet printing. Specifically, by using DPP 2831 (manufactured by FUJIFILM Dimatix, Inc.) as an ink jet device and a 10 pL head, a solid film was formed at a jetting frequency of 2 Hz and a dot pitch of 20 μm. Then, the film was dried for 1 hour at 70°C such that an organic semiconductor layer was formed, thereby obtaining a bottom gate-bottom contact type organic TFT element.

In addition, the composition for forming an organic semiconductor film was applied to the substrate by flexographic printing. Specifically, as a printing device, a flexographic printability tester F1 (manufactured by IGT Testing Systems K.K.) was used, and as a flexographic resin plate, a plate-like photosensitive resin AFP DSH 1.70% (manufactured by Asahi Kasei Corporation.)/solid image was used. Printing was performed at a transport rate of 0.4 m/sec

with applying a pressure of 60 N between the plate and the substrate, and then the substrate was dried as it was for 2 hours at room temperature of equal to or lower than 40°C such that an organic semiconductor layer was formed, thereby obtaining a bottom gate-bottom contact type organic TFT element.

<Characteristic evaluation>

By using a semiconductor characteristic evaluation device B2900A (manufactured by Agilent Technologies), the performance of the elements was evaluated as below in the atmosphere.

(a) Carrier mobility

A voltage of -60 V was appilied between the source electrode and the drain electrode of each of the organic TFT elements, a gate voltage was varied within a range of +10 V to -60 V, and a carrier moblity $\mu$ was calcuated using the following equation showing a drain current $I_d$.

$$I_d = (w/2L)\mu C_i(V_g - V_{th})^2$$

In the equation, L represents a gate length, W represents a gate width, $C_i$ represents a capacity of the insulating layer per unit area, $V_g$ represents a gate voltage, and $V_{th}$ represents a threshold voltage.

The higher the carrier mobility $\mu$, the more preferable. For practical use, the carrier mobility is preferably equal to or greater than $1 \times 10^{-2}$ $cm^2/Vs$, and more preferably equal to or greater than $1 \times 10^{-1}$ $cm^2/Vs$.

(b) Threshold voltage shift after repeated driving (threshold voltage shift)

Between the source electrode and the drain electrode of each of the organic TFT elements, a voltage of -60 V was applied, and the element was repeatedly driven 500 times by varying the gate voltage within a range of +10 V to -60 V. In this way, the element was measured in the same manner as in (a), and a difference between a threshold voltage $V_{before}$ before the repeated driving and a threshold voltage $V_{after}$ after the repeated driving ($|V_{after}-V_{before}|$) was evaluated into 4 levels as below. The smaller the difference, the higher the stability of the element against repeated driving. Therefore, the smaller the difference, the more preferable. For practical use, the difference is preferably S or A, and more preferably S. S:

$$|V_{after} - V_{before}| \leq 2\ V$$

A:

$$2\ V < |V_{after} - V_{before}| \leq 3\ V$$

B:

$$3\ V < |V_{after} - V_{before}| \leq 6\ V$$

C:

$$6\ V < |V_{after} - V_{before}| \leq 9\ V$$

D:

$$|V_{after} - V_{before}| > 9\ V$$

(c) Mobility retention rate in case where coating solution having undergone cold preservation (mobility retention rate)

Organic TFT elements were prepared in the same manner as described above, except that a composition for forming an organic semiconductor film was used which was cold-preserved for 7 days at 0°C in a capped state, then returned to room temperature, and filtered through a 0.5 μm membrane filter. Then, a carrier mobility $\mu_{cold}$ determined by calculating a carrier mobility in the same manner as in (a) was divided by a carrier mobility μ measured by a common method, and the obtained value ($\mu_{cold}/\mu$) was evaluated into 5 levels as below. The greater the value, the higher the preservation stability of the coating solution. For practical use, the value is preferably S, A, or B, more preferably S or A, and particularly preferably S. S:

$$\mu_{cold}/\mu > 0.95$$

A:

$$0.70 < \mu_{cold}/\mu \leq 0.95$$

B:

$$0.50 < \mu_{cold}/\mu \leq 0.70$$

C:

$$0.10 < \mu_{cold}/\mu \leq 0.50$$

D:

$$\mu_{cold}/\mu \leq 0.10$$

The following Table 1 shows the results obtained in a case where the composition for forming an organic semiconductor film was applied by spin coating method. Table 2 shows the results obtained in a case where the composition for forming an organic semiconductor film was applied by ink jet printing or flexographic printing.

[Table 1]

| | Element No. | Organic semiconductor | Binder polymer | Solvent | Concentration Organic semiconductor/binder polymer (mass%) | Carrier mobility ($cm^2/Vs$) | Threshold voltage shift | Mobility retention rate |
|---|---|---|---|---|---|---|---|---|
| Example 1* | Element 1-1 | Compound 1 | - | Tetralin | 1.5 | $4.9 \times 10^{-2}$ | S | A |
| Example 2* | Element 1-2 | Compound 2 | - | Tetralin | 1.0 | $4.4 \times 10^{-2}$ | S | A |
| Example 3* | Element 1-3 | Compound 2 | PTAA | Tetralin | 1.0/0.5 | $4.3 \times 10^{-2}$ | S | A |
| Example 4* | Element 1-4 | Compound 3 | - | Tetralin | 1.5 | $6.3 \times 10^{-2}$ | S | A |
| Example 5* | Element 1-5 | Compound 3 | PαMS | Tetralin | 1.5/1.5 | $7.2 \times 10^{-2}$ | S | A |
| Example 6* | Element 1-6 | Compound 4 | - | Mesitylene | 1.0 | $9.3 \times 10^{-2}$ | S | A |
| Example 7* | Element 1-7 | Compound 4 | - | Cyclohexanone | 1.0 | $7.9 \times 10^{-2}$ | S | A |
| Example 8 | Element 1-8 | Compound 5 | - | Diethylbenzene | 1.0 | $2.1 \times 10^{-1}$ | S | A |
| Example 9* | Element 1-9 | Compound 6 | - | Mesitylene | 2.0 | $1.6 \times 10^{-1}$ | S | A |
| Example 10* | Element 1-10 | Compound 6 | PCPDTBT | Mesitylene | 2.0/0.50 | $1.6 \times 10^{-1}$ | S | A |
| Example 11* | Element 1-11 | Compound 7 | - | Anisole | 1.0 | $1.3 \times 10^{-2}$ | A | A |
| Example 12* | Element 1-12 | Compound 8 | - | Tetralin | 1.0 | $1.0 \times 10^{-1}$ | S | A |
| Example 13 | Element 1-13 | Compound 9 | - | Anisole | 1.0 | $7.7 \times 10^{-2}$ | S | A |
| Example 14 | Element 1-14 | Compound 9 | - | Tetralin | 1.0 | $7.8 \times 10^{-2}$ | S | A |

(continued)

| | Element No. | Organic semiconductor | Binder polymer | Solvent | Concentration Organic semiconductor/binder polymer (mass%) | Carrier mobility ($cm^2/Vs$) | Threshold voltage shift | Mobility retention rate |
|---|---|---|---|---|---|---|---|---|
| Example 15* | Element 1-15 | Compound 10 | - | Anisole | 1.0 | $2.4 \times 10^{-1}$ | S | A |
| Example 16* | Element 1-16 | Compound 10 | - | Anisole | 5.0 | $3.6 \times 10^{-1}$ | S | A |
| Example 17* | Element 1-17 | Compound 10 | - | Anisole | 7.5 | $3.4 \times 10^{-1}$ | S | A |
| Example 18* | Element 1-18 | Compound 11 | - | Tetralin | 1.5 | $4.0 \times 10^{-2}$ | A | A |
| Example 19* | Element 1-19 | Compound 12 | - | Tetralin | 1.0 | $5.1 \times 10^{-2}$ | S | A |
| Example 20* | Element 1-20 | Compound 12 | - | N-methylpyrrolidone | 1.0 | $3.3 \times 10^{-2}$ | S | A |
| Example 21* | Element 1-21 | Compound 13 | - | Tetralin | 1.0 | $3.5 \times 10^{-2}$ | S | A |
| Example 22* | Element 1-22 | Compound 14 | - | Anisole | 0.7 | $1.4 \times 10^{-1}$ | S | A |
| Example 23* | Element 1-23 | Compound 14 | - | Tetralin | 0.7 | $1.4 \times 10^{-1}$ | S | A |
| Example 24* | Element 1-24 | Compound 14 | - | Tetralin | 3.0 | $2.3 \times 10^{-1}$ | S | A |
| Example 25* | Element 1-25 | Compound 15 | - | Anisole | 1.0 | $9.1 \times 10^{-2}$ | S | A |
| Example 26* | Element 1-26 | Compound 15 | P$\alpha$MS | Anisole | 1.0/0.75 | $9.7 \times 10^{-2}$ | S | A |
| Example 27* | Element 1-27 | Compound 16 | - | Anisole | 0.7 | $9.8 \times 10^{-2}$ | S | A |
| Example 28* | Element 1-28 | Compound 16 | PCPDTBT | Anisole | 0.7/0.7 | $9.9 \times 10^{-2}$ | S | A |

(continued)

| | Element No. | Organic semiconductor | Binder polymer | Solvent | Concentration Organic semiconductor/binder polymer (mass%) | Carrier mobility ($cm^2/Vs$) | Threshold voltage shift | Mobility retention rate |
|---|---|---|---|---|---|---|---|---|
| Example 29* | Element 1-29 | Compound 17 | - | Tetralin | 1.0 | $7.2 \times 10^{-2}$ | A | A |
| Example 30* | Element 1-30 | Compound 18 | - | Tetralin | 1.0 | $2.9 \times 10^{-2}$ | S | A |
| Example 31* | Element 1-31 | Compound 18 | PTAA | Tetralin | 1.0/1.0 | $3.3 \times 10^{-2}$ | S | A |
| Example 32* | Element 1-32 | Compound 19 | - | Anisole | 1.0 | $1.8 \times 10^{-2}$ | A | A |
| Example 33* | Element 1-33 | Compound 10 | - | Ani sole/ chlorobenzene = 2/1 | 1.0 | $1.9 \times 10^{-1}$ | A | A |
| Comparative Example 1 | Element 1-34 | Compound 10 | - | Ani sole/ chlorobenzene = 1/2 | 1.0 | $2.3 \times 10^{-1}$ | C | A |
| Comparative Example 2 | Element 1-35 | Compound 10 | - | Chloroform | 1.0 | $9.4 \times 10^{-2}$ | D | A |
| Comparative Example 3 | Element 1-36 | Compound 10 | - | Chlorobenzene | 1.0 | $2.0 \times 10^{-1}$ | D | A |
| Comparative Example 4 | Element 1-37 | Compound 14 | - | Tetralin | 0.5 | $2.9 \times 10^{-2}$ | B | A |
| Comparative Example 5 | Element 1-38 | Compound 14 | - | Tetralin | 0.3 | $1.6 \times 10^{-2}$ | B | A |
| Comparative Example 6 | Element 1-39 | Comparative compound 1 | - | Anisole | 1.0 | $3.5 \times 10^{-5}$ | D | B |
| Comparative Example 7 | Element 1-40 | Comparative compound 2 | - | Anisole | 1.0 | $1.9 \times 10^{-5}$ | D | B |
| Comparative Example 8 | Element 1-41 | Comparative compound 3 | - | Tetralin | 1.0 | $8.2 \times 10^{-5}$ | B | C |
| Comparative Example 9 | Element 1-42 | Comparative compound 4 | - | Anisole | 1.0 | $1.7 \times 10^{-4}$ | B | D |

(continued)

| | Element No. | Organic semiconductor | Binder polymer | Solvent | Concentration Organic semiconductor/binder polymer (mass%) | Carrier mobility ($cm^2/Vs$) | Threshold voltage shift | Mobility retention rate |
|---|---|---|---|---|---|---|---|---|
| Comparative Example 10 | Element 1-43 | Comparative compound 5 | - | Anisole | 1.0 | $7.3 \times 10^{-4}$ | D | D |
| Comparative Example 11 | Element 1-44 | Comparative compound 6 | - | Tetralin | 1.0 | $2.2 \times 10^{-4}$ | C | C |
| Comparative Example 12 | Element 1-45 | Comparative compound 6 | - | Tetralin | 2.0 | $2.9 \times 10^{-4}$ | D | D |
| Comparative Example 13 | Element 1-46 | Comparative compound 7 | - | Anisole | 0.5 | $1.1 \times 10^{-2}$ | B | D |
| Comparative Example 14 | Element 1-47 | Comparative compound 8 | - | Anisole | 1.0 | $2.7 \times 10^{-5}$ | D | C |
| *: Reference Examples | | | | | | | | |

[Table 2]

| | Element No. | Organic semiconductor | Binder polymer | Solvent | Concentration Organic semiconductor/binder polymer (mass%) | Application method | Carrier mobility ($cm^2/Vs$) | Threshold voltage shift |
|---|---|---|---|---|---|---|---|---|
| Example 34* | Element 2-1 | Compound 1 | PCPDTBT | Anisole | 1.0/1.0 | Ink j et | $7.1 \times 10^{-2}$ | S |
| Example 35* | Element 2-2 | Compound 2 | PCPDTBT | Tetraln | 2.0/2.0 | Flexography | $5.8 \times 10^{-2}$ | S |
| Example 36* | Element 2-3 | Compound 3 | - | Toluene | 2.0 | Ink jet | $8.1 \times 10^{-2}$ | s |
| Example 37* | Element 2-4 | Compound 3 | - | Mesitylene | 2.0 | Ink j et | $9.6 \times 10^{-2}$ | S |
| Example 38* | Element 2-5 | Compound 3 | - | Anisole | 2.0 | Ink j et | $1.2 \times 10^{-1}$ | S |
| Example 39* | Element 2-6 | Compound 3 | - | Tetralin | 2.0 | Ink jet | $1.3 \times 10^{-1}$ | s |
| Example 40* | Element 2-7 | Compound 4 | - | Tetralin | 5.0 | Ink j et | $3.3 \times 10^{1}$ | S |
| Example 41 | Element 2-8 | Compound 5 | PCPDTBT | Tetralin | 1.0/0.5 | Ink j et | $5.1 \times 10^{-1}$ | S |
| Example 42 | Element 2-9 | Compound 5 | PCPDTBT | Tetralin | 1.5/0.5 | Ink jet | $6.2 \times 10^{-1}$ | s |
| Example 43* | Element 2-10 | Compound 6 | PαMS | Tetralin | 7.5/2.5 | Flexography | $1.5 \times 10^{-1}$ | S |
| Example 44* | Element 2-11 | Compound 10 | PαMS | Tetralin | 2.0/2.0 | Flexography | $4.7 \times 10^{-1}$ | S |
| Example 45* | Element 2-12 | Compound 12 | PCPDTBT | Tetralin | 2.0/0.5 | Ink jet | $6.8 \times 10^{-2}$ | s |
| Example 46* | Element 2-13 | Compound 14 | PαMS | Anisole | 1.0/0.5 | Flexography | $2.4 \times 10^{-1}$ | S |
| Example 47* | Element 2-14 | Compound 14 | PαMS | Anisole | 1.0/1.0 | Flexography | $3.0 \times 10^{-1}$ | S |

(continued)

| | Element No. | Organic semiconductor | Binder polymer | Solvent | Concentration Organic semiconductor/binder polymer (mass%) | Application method | Carrier mobility ($cm^2/Vs$) | Threshold voltage shift |
|---|---|---|---|---|---|---|---|---|
| Example 48* | Element 2-15 | Compound 15 | PTAA | Tetralin | 1.0/1.0 | Ink jet | $3.5 \times 10^{-1}$ | s |
| Example 49* | Element 2-16 | Compound 17 | PCPDTBT | Tetralin | 1.5/0.50 | Ink j et | $1.2 \times 10^{-1}$ | S |
| Example 50* | Element 2-17 | Compound 18 | PTAA | Tetralin | 2.5/2.0 | Flexography | $4.1 \times 10^{-2}$ | S |
| *: Reference Examples | | | | | | | | |

As shown in Tables 1 and 2, it was confirmed that the composition for forming an organic semiconductor film of the present invention has excellent preservation stability, makes the obtained organic semiconductor exhibit excellent driving stability in the atmosphere, and has high carrier mobility.

In contrast, the compositions for forming an organic semiconductor film of comparative examples failed to achieve both of the preservation stability and the performance of making the obtained organic semiconductor exhibit driving stability in the atmosphere. Explanation of References

| | |
|---|---|
| 10: | substrate |
| 20: | gate electrode |
| 30: | gate insulating film |
| 40: | source electrode |
| 42: | drain electrode |
| 50: | organic semiconductor film |
| 51: | metal mask |
| 52: | mask portion |
| 53, 54: | opening portion |
| 60: | sealing layer |
| 100, 200: | organic thin film transistor |

**Claims**

1. A composition for forming an organic semiconductor film, comprising:

an organic semiconductor represented by the following Formula A-2 as a component A;
a solvent as a component B; and
optionally a binder polymer as a component C,
wherein a content of a non-halogen-based solvent is equal to or greater than 50% by mass with respect to a total content of the component B, and
a content of the component A in the composition for forming an organic semiconductor film is equal to or greater than 0.7% by mass and less than 15% by mass,

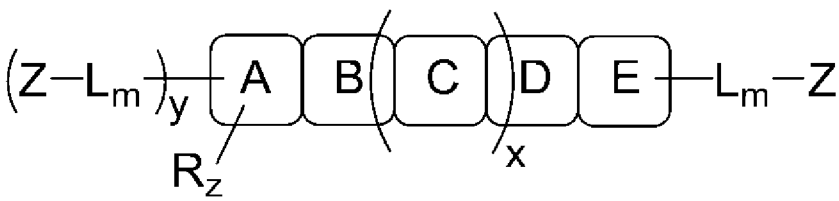

(A - 2)

in Formula A-2, rings A to E each independently represent a benzene ring or a thiophene ring, and preferably 2 to 4 rings among the rings A to E are thiophene rings; R represents an alkyl group having 1 to 20 carbon atoms, an alkenyl group having 2 to 20 carbon atoms, an alkynyl group having 2 to 20 carbon atoms, an aromatic hydrocarbon group having 6 to 30 carbon atoms, an aromatic heterocyclic group which is a 5- to 30-membered ring, or a fluorine atom; L each independently represents a phenylene group or a thienylene group; Z each independently represents a group represented by Formula a-1; m each independently represents an integer of 1 to 4; when there are two or more L's, L's may be the same as or different from each other; when there are two or more Z's, Z's may be the same as or different from each other; x represents an integer of 1 to 3; y represents 0 or 1; z represents 0 or 1; and the symmetry of a ring-fused structure formed of the rings A to E is $C_2$, $C_{2v}$, or $C_{2h}$,,

$$*-(CH_2)_p-O-(CH_2)_q-CH_3 \qquad (a-1)$$

in Formula a-1, p represents an integer of 1 to 20, and preferably an integer of 1 to 6, q represents an integer

of 0 to 20, and * represents a binding position with respect to other structures, wherein the rings A and E are thiophene rings and/or L is a thienylene group.

**2.** The composition for forming an organic semiconductor film according to claim 1, wherein a boiling point of the non-halogen-based solvent is equal to or higher than 100°C.

**3.** The composition for forming an organic semiconductor film according to claim 1 or 2, wherein the non-halogen-based solvent contains an aromatic solvent in an amount of equal to or greater than 50% by mass.

**4.** The composition for forming an organic semiconductor film according to any one of claims 1 to 3 that has a viscosity, as determined by a method based on JIS Z8803, of equal to or higher than 5 mPa·s and equal to or lower than 40 mPa·s at 25°C.

**5.** The composition for forming an organic semiconductor film according to any one of claims 1 to 4, wherein a concentration of total solid contents is equal to or higher than 1.5% by mass.

**6.** The composition for forming an organic semiconductor film according to any one of claims 1 to 5 that is used for ink jet printing and/or flexographic printing.

**7.** A method for manufacturing an organic semiconductor film, comprising:

an application step of applying the composition for forming an organic semiconductor film according to any one of claims 1 to 6 onto a substrate; and
a drying step of removing a solvent from the applied composition.

**8.** A method for manufacturing an organic semiconductor element, the method comprising the manufacture of an organic semiconductor film according to the method of claim 7.

**9.** The method for manufacturing an organic semiconductor element according to claim 8, wherein the application step is performed by ink jet printing or flexographic printing.

**10.** An organic semiconductor compound represented by Formula A-2,

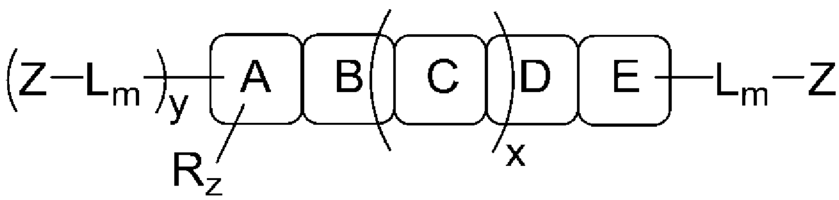

(A-2)

in Formula A-2, rings A to E each independently represent a benzene ring or a thiophene ring, and preferably 2 to 4 rings among the rings A to E are thiophene rings; R represents an alkyl group having 1 to 20 carbon atoms, an alkenyl group having 2 to 20 carbon atoms, an alkynyl group having 2 to 20 carbon atoms, an aromatic hydrocarbon group having 6 to 30 carbon atoms, an aromatic heterocyclic group which is 5- to 30-membered ring, or a fluorine atom; L each independently represents a phenylene group or a thienylene group; Z each independently represents a group represented by the following Formula a-1; m each independently represents an integer of 1 to 4; when there are two or more L's, L's may be the same as or different from each other; when there are two or more Z's, Z's may be the same as or different from each other; x represents an integer of 1 to 3; y represents 0 or 1; z represents 0 or 1; and the symmetry of a ring-fused structure formed of the rings A to E is $C_2$, $C_{2v}$, or $C_{2h}$,

$$*-(CH_2)_p-O-(CH_2)_q-CH_3 \quad (a-1)$$

in Formula a-1, p represents an integer of 1 to 20, and preferably an integer of 1 to 6, q represents an integer of 0

to 20, and * represents a binding position with respect to other structures,
wherein the rings A and E are thiophene rings and/or L is a thienylene group.

**11.** An organic semiconductor film comprising the organic semiconductor compound according to claim 10.

**12.** An organic semiconductor element comprising the organic semiconductor film according to claim 11.

**Patentansprüche**

**1.** Zusammensetzung zur Bildung einer organischen Halbleiterschicht, umfassend:

einen organischen Halbleiter, der durch die folgende Formel A-2 als Komponente A dargestellt wird;
ein Lösungsmittel als Komponente B; und
optional ein Bindemittelpolymer als Komponente C,
wobei ein Gehalt eines Lösungsmittels auf halogenfreier Basis gleich oder größer als 50 Massenprozent in Bezug auf den Gesamtgehalt der Komponente B beträgt, und
ein Gehalt der Komponente A in der Zusammensetzung zur Bildung einer organischen Halbleiterschicht gleich oder größer als 0,7 Massenprozent und kleiner als 15 Massenprozent beträgt,

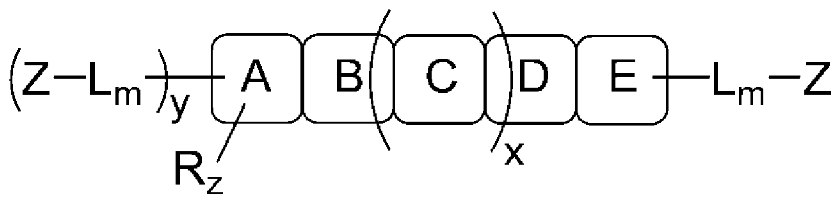

(A-2)

worin in der Formel A-2 Ringe A bis E jeweils unabhängig voneinander einen Benzolring oder einen Thiophenring darstellen, und vorzugsweise 2 bis 4 Ringe unter den Ringen A bis E Thiophenringe sind; R eine Alkylgruppe mit 1 bis 20 Kohlenstoffatomen, eine Alkenylgruppe mit 2 bis 20 Kohlenstoffatomen, eine Alkinylgruppe mit 2 bis 20 Kohlenstoffatomen, eine aromatische Kohlenwasserstoffgruppe mit 6 bis 30 Kohlenstoffatomen, eine aromatische heterocyclische Gruppe, die ein 5- bis 30-gliedriger Ring ist, oder ein Fluoratom darstellt; L jeweils unabhängig voneinander eine Phenylengruppe oder eine Thienylengruppe darstellt; Z jeweils unabhängig voneinander eine durch die Formel a-1 dargestellte Gruppe darstellt; m jeweils unabhängig voneinander eine ganze Zahl von 1 bis 4 darstellt; wenn zwei oder mehrere L vorhanden sind, können die L gleich oder verschieden voneinander sein; wenn zwei oder mehrere Z vorhanden sind, können die Z gleich oder verschieden voneinander sein; x eine ganze Zahl von 1 bis 3 darstellt; y 0 oder 1 darstellt; z 0 oder 1 darstellt; und die Symmetrie einer aus den Ringen A bis E gebildeten ringkondensierten Struktur $C_2$, $C_{2v}$ oder $C_{2h}$ ist,

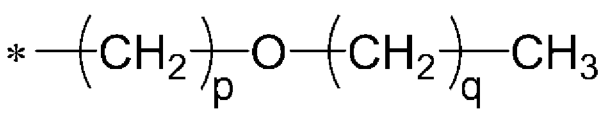

(a-1)

worin in der Formel a-1 p eine ganze Zahl von 1 bis 20 und vorzugsweise eine ganze Zahl von 1 bis 6 darstellt, q eine ganze Zahl von 0 bis 20 darstellt, und * eine Bindungsposition in Bezug auf andere Strukturen darstellt,
worin die Ringe A und E Thiophenringe sind und/oder L eine Thienylengruppe ist.

**2.** Zusammensetzung zur Bildung einer organischen Halbleiterschicht gemäß Anspruch 1,
wobei der Siedepunkt des Lösungsmittels auf halogenfreier Basis gleich oder höher als 100 °C ist.

**3.** Zusammensetzung zur Bildung einer organischen Halbleiterschicht gemäß Anspruch 1 oder 2,
wobei das Lösungsmittel auf halogenfreier Basis ein aromatisches Lösungsmittel in einer Menge von gleich oder mehr als 50 Massenprozent enthält.

**4.** Zusammensetzung zur Bildung einer organischen Halbleiterschicht gemäß mindestens einem der Ansprüche 1 bis 3, die eine Viskosität, bestimmt durch ein Verfahren auf der Grundlage von JIS Z8803, von gleich oder höher als 5

mPa·s und gleich oder niedriger als 40 mPa·s bei 25 °C aufweist.

5. Zusammensetzung zur Bildung einer organischen Halbleiterschicht gemäß mindestens einem der Ansprüche 1 bis 4, wobei die Konzentration des gesamten Feststoffgehalts gleich oder höher als 1,5 Massenprozent ist.

6. Zusammensetzung zur Bildung einer organischen Halbleiterschicht gemäß mindestens einem der Ansprüche 1 bis 5, die für den Tintenstrahldruck und/oder den Flexodruck verwendet wird.

7. Verfahren zur Herstellung einer organischen Halbleiterschicht, umfassend:

   einen Auftragungsschritt bei dem die Zusammensetzung zur Bildung einer organischen Halbleiterschicht gemäß mindestens einem der Ansprüche 1 bis 6 auf ein Substrat aufgetragen wird; und
   einen Trocknungsschritt bei dem Lösungsmittel aus der aufgetragenen Zusammensetzung entfernt wird.

8. Verfahren zur Herstellung einer organischen Halbleiterschicht, wobei das Verfahren die Herstellung einer organischen Halbleiterschicht gemäß dem Verfahren nach Anspruch 7 umfasst.

9. Verfahren zur Herstellung einer organischen Halbleiterschicht gemäß Anspruch 8,
   wobei der Auftragungsschritt durch Tintenstrahldruck oder Flexodruck durchgeführt wird.

10. Organische Halbleiterverbindung, dargestellt durch die Formel A-2,

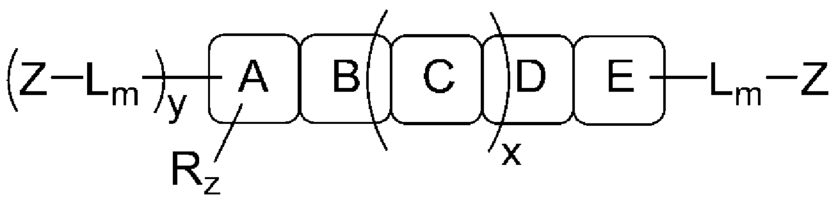

(A - 2)

   worin in der Formel A-2 Ringe A bis E jeweils unabhängig voneinander einen Benzolring oder einen Thiophenring darstellen, und vorzugsweise 2 bis 4 Ringe unter den Ringen A bis E Thiophenringe sind; R eine Alkylgruppe mit 1 bis 20 Kohlenstoffatomen, eine Alkenylgruppe mit 2 bis 20 Kohlenstoffatomen, eine Alkinylgruppe mit 2 bis 20 Kohlenstoffatomen, eine aromatische Kohlenwasserstoffgruppe mit 6 bis 30 Kohlenstoffatomen, eine aromatische heterocyclische Gruppe, die ein 5- bis 30-gliedriger Ring ist, oder ein Fluoratom darstellt; L jeweils unabhängig voneinander eine Phenylengruppe oder eine Thienylengruppe darstellt; Z jeweils unabhängig voneinander eine durch die folgende Formel a-1 dargestellte Gruppe darstellt; m jeweils unabhängig voneinander eine ganze Zahl von 1 bis 4 darstellt; wenn zwei oder mehrere L vorhanden sind, können die L gleich oder verschieden voneinander sein; wenn zwei oder mehrere Z vorhanden sind, können die Z gleich oder verschieden voneinander sein; x eine ganze Zahl von 1 bis 3 darstellt; y 0 oder 1 darstellt; z 0 oder 1 darstellt; und die Symmetrie einer aus den Ringen A bis E gebildeten ringkondensierten Struktur $C_2$, $C_{2v}$ oder $C_{2h}$ ist,

$$*-(CH_2)_p-O-(CH_2)_q-CH_3 \qquad (a-1)$$

   worin in der Formel a-1 p eine ganze Zahl von 1 bis 20 und vorzugsweise eine ganze Zahl von 1 bis 6 darstellt, q eine ganze Zahl von 0 bis 20 darstellt, und * eine Bindungsposition in Bezug auf andere Strukturen darstellt, worin die Ringe A und E Thiophenringe sind und/oder L eine Thienylengruppe ist.

11. Organische Halbleiterschicht, umfassend die organische Halbleiterverbindung gemäß Anspruch 10.

12. Organisches Halbleiterelement umfassend die organische Halbleiterschicht gemäß Anspruch 11.

## Revendications

1. **1.** Composition pour former un film de semi-conducteur organique, comprenant :

    un semi-conducteur organique représenté par la formule A-2 suivante comme composant A ;
    un solvant comme composant B ; et
    facultativement un polymère liant comme composant C,
    dans laquelle une teneur d'un solvant non à base d'halogène est égale ou supérieure à 50 % en masse par rapport à une teneur totale du composant B, et
    une teneur du composant A dans la composition pour former un film de semi-conducteur organique est égale ou supérieure à 0,7 % en masse et inférieure à 15 % en masse,

    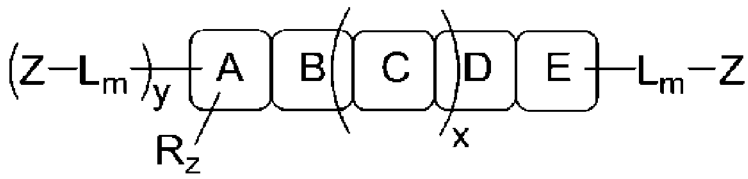

    (A-2)

    dans la formule A-2, les cycles A à E représentent chacun indépendamment un cycle benzène ou un cycle thiophène, et de préférence, de 2 à 4 cycles parmi les cycles A à E sont des cycles thiophène ; R représente un groupe alkyle présentant de 1 à 20 atomes de carbone, un groupe alcényle présentant de 2 à 20 atomes de carbone, un groupe alcynyle présentant de 2 à 20 atomes de carbone, un groupe hydrocarboné aromatique présentant de 6 à 30 atomes de carbone, un groupe hétérocyclique aromatique qui est un cycle à 5 à 30 chaînons, ou un atome de fluor; L représente chacun indépendamment un groupe phénylène ou un groupe thiénylène ; Z représente chacun indépendamment un groupe représenté par la formule a-1 ; m représente chacun indépendamment un nombre entier de 1 à 4 ; lorsqu'il y a deux L ou plus, les L peuvent être identiques ou différents les uns des autres ; lorsqu'il y a deux Z ou plus, les Z peuvent être identiques ou différents les uns des autres ; x représente un nombre entier de 1 à 3 ; y représente 0 ou 1 ; z représente 0 ou 1 ; et la symétrie d'une structure à cycles fusionnés formée des cycles A à E est C2, C2v ou C2h,

    $$*\text{—}(CH_2)_p\text{—}O\text{—}(CH_2)_q\text{—}CH_3 \qquad (a-1)$$

    dans la formule a-1, p représente un nombre entier de 1 à 20, et de préférence un nombre entier de 1 à 6, q représente un nombre entier de 0 à 20, et * représente une position de liaison par rapport à d'autres structures, dans laquelle les cycles A et E sont des cycles thiophène et/ou L est un groupe thiénylène.

2. **2.** Composition pour former un film de semi-conducteur organique selon la revendication 1, dans laquelle un point d'ébullition du solvant non à base d'halogène est égal ou supérieur à 100 °C.

3. **3.** Composition pour former un film de semi-conducteur organique selon la revendication 1 ou 2, dans laquelle le solvant non à base d'halogène contient un solvant aromatique dans une quantité égale ou supérieure à 50 % en masse.

4. **4.** Composition pour former un film de semi-conducteur organique selon l'une quelconque des revendications 1 à 3 qui présente une viscosité, telle que déterminée par un procédé basé sur JIS Z8803, égale ou supérieure à 5 mPa.s et égale ou inférieure à 40 mPa.s à 25°C.

5. **5.** Composition pour former un film de semi-conducteur organique selon l'une quelconque des revendications 1 à 4, dans laquelle une concentration des teneurs totales en solides est égale ou supérieure à 1,5 % en masse.

6. **6.** Composition pour former un film de semi-conducteur organique selon l'une quelconque des revendications 1 à 5 qui est utilisée pour une impression à jet d'encre et/ou une impression flexographique.

7. **7.** Procédé de fabrication d'un film de semi-conducteur organique, comprenant :

une étape d'application consistant à appliquer la composition pour former un film de semi-conducteur organique selon l'une quelconque des revendications 1 à 6 sur un substrat ; et
une étape de séchage consistant à retirer un solvant de la composition appliquée.

**8.** Procédé de fabrication d'un élément de semi-conducteur organique, le procédé comprenant la fabrication d'un film de semi-conducteur organique selon le procédé de la revendication 7.

**9.** Procédé de fabrication d'un élément de semi-conducteur organique selon la revendication 8,
dans lequel l'étape d'application est effectuée par impression à jet d'encre ou impression flexographique.

**10.** Composé de semi-conducteur organique représenté par la formule A-2,

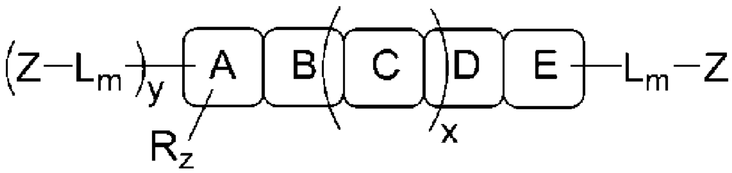

(A-2)

dans la formule A-2, les cycles A à E représentent chacun indépendamment un cycle benzène ou un cycle thiophène, et de préférence de 2 à 4 cycles parmi les cycles A à E sont des cycles thiophène ; R représente un groupe alkyle présentant de 1 à 20 atomes de carbone, un groupe alcényle présentant de 2 à 20 atomes de carbone, un groupe alcynyle présentant de 2 à 20 atomes de carbone, un groupe hydrocarboné aromatique présentant de 6 à 30 atomes de carbone, un groupe hétérocyclique aromatique qui est un cycle à 5 à 30 chaînons, ou un atome de fluor; L représente chacun indépendamment un groupe phénylène ou un groupe thiénylène ; Z représente chacun indépendamment un groupe représenté par la formule a-1 suivante ; m représente chacun indépendamment un nombre entier de 1 à 4; lorsqu'il y a deux L ou plus, les L peuvent être identiques ou différents les uns des autres ; lorsqu'il y a deux Z ou plus, les Z peuvent être identiques ou différents les uns des autres ; x représente un nombre entier de 1 à 3 ; y représente 0 ou 1; z représente 0 ou 1 ; et la symétrie d'une structure à cycles fusionnés formée des cycles A à E est $C_2$, $C_{2v}$, ou $C_{2h}$,

$$* \text{—}(CH_2)_p\text{—}O\text{—}(CH_2)_q\text{—}CH_3 \qquad (a-1)$$

dans la formule a-1, p représente un nombre entier de 1 à 20, et de préférence un nombre entier de 1 à 6, q représente un nombre entier de 0 à 20, et * représente une position de liaison par rapport à d'autres structures, dans laquelle les cycles A et E sont des cycles thiophène et/ou L est un groupe thiénylène.

**11.** Film de semi-conducteur organique comprenant le composé de semi-conducteur organique selon la revendication 10.

**12.** Élément de semi-conducteur organique comprenant le film de semi-conducteur organique selon la revendication 11.

FIG. 1

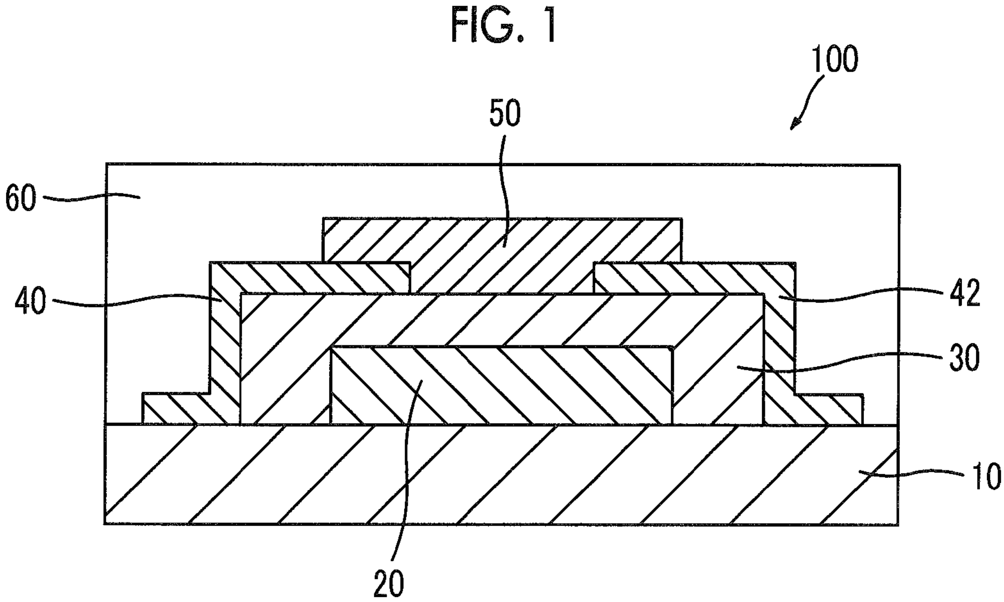


FIG. 2

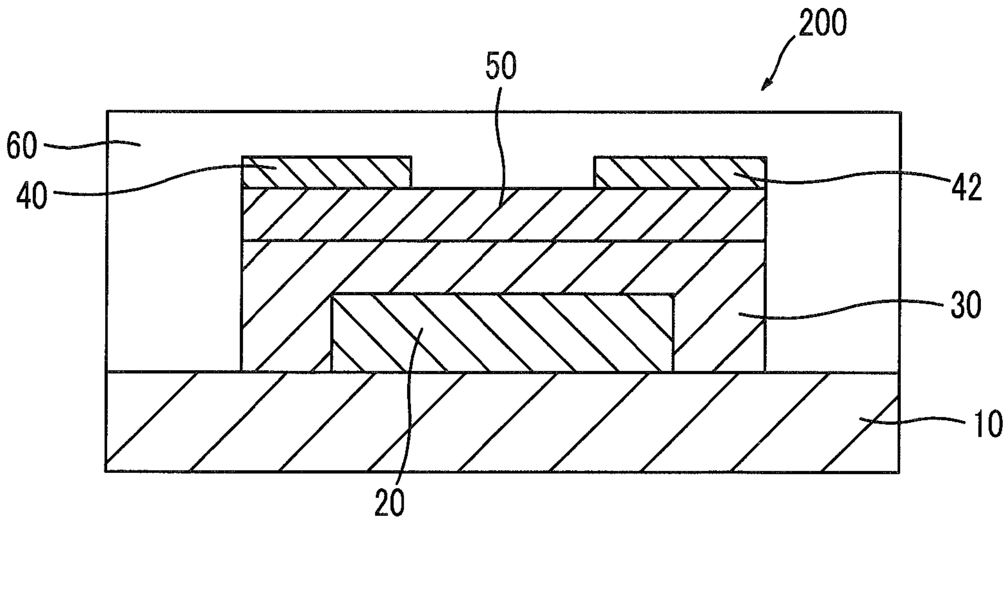

FIG. 3

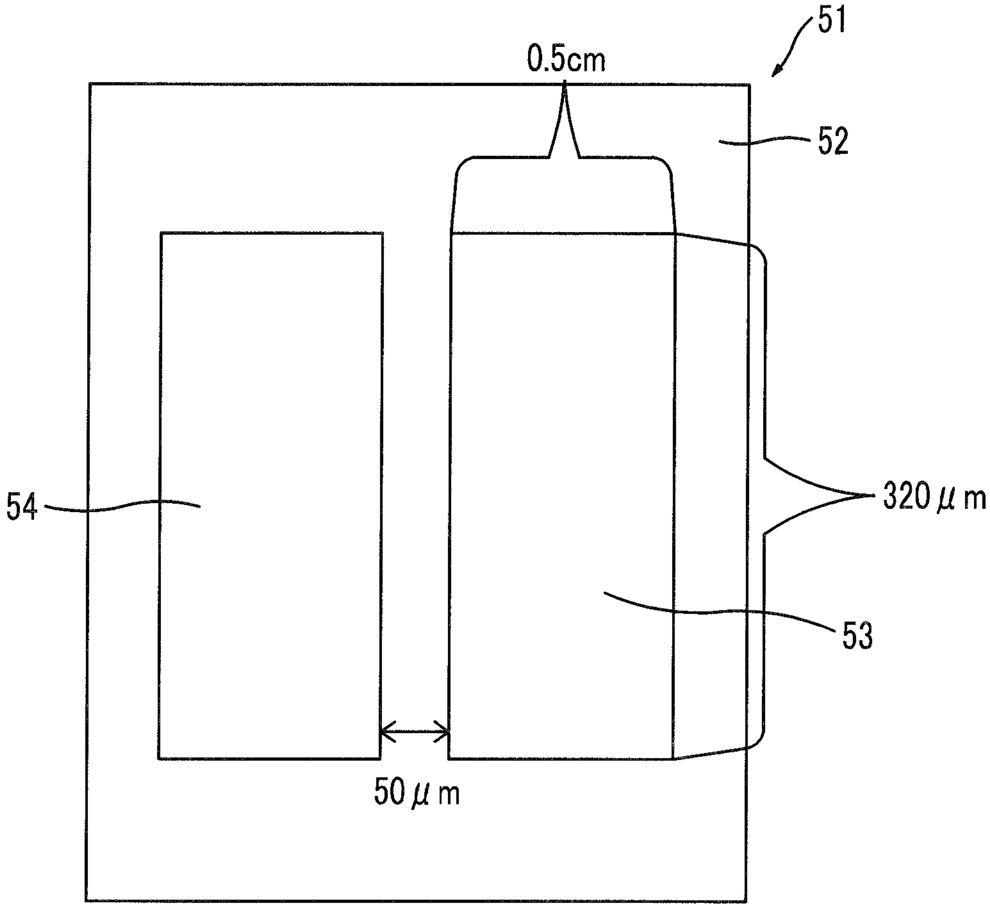

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2009267134 A
- JP 5352260 B
- JP 2009267140 A
- US 5936259 A1
- JP 2010177642 A
- JP 2009267132 A
- JP 2012510454 A
- JP 2013191821 A
- JP 2009246140 A
- JP 2011032268 A
- JP 2009054810 A
- JP 2011526588 A
- JP 2010520241 A
- JP 2010006794 A
- JP 2006176491 A
- US 20080142792 A
- WO 2010098372 A


### Non-patent literature cited in the description


- *Adv. Mater.,* 2013, vol. 25, 6392
- *Chem. Commun.,* 2014, vol. 50, 5342
- *Appl. Phys. Express,* 2013, vol. 6, 076503
- *Scientific Reports,* 2014, vol. 4, 5048